(19) Europäisches Patentamt
European Patent Office
Office européen des brevets

(11) Publication number: 0 274 425 B1

(12) **EUROPEAN PATENT SPECIFICATION**

(45) Date of publication of patent specification: **15.12.93**

(51) Int. Cl.5: **C12N 15/53**, C12N 9/02, C12Q 1/00, C12Q 1/48, C12Q 1/26

(21) Application number: **88300073.9**

(22) Date of filing: **06.01.88**

# (54) Pyruvate oxidase, its preparation and use.

(30) Priority: **06.01.87 JP 903/87**
**15.05.87 JP 118161/87**

(43) Date of publication of application:
**13.07.88 Bulletin 88/28**

(45) Publication of the grant of the patent:
**15.12.93 Bulletin 93/50**

(84) Designated Contracting States:
**DE FR IT**

(56) References cited:
**EP-A- 0 117 550**
**FR-A- 2 420 760**
**FR-A- 2 436 182**

**NUCLEIC ACID RESEARCH, vol. 14, no. 13, 1986, pages 5449-5460; C. GRABAU et al.: "Nucleotide sequence and deduced amino acid sequence of Escherichia coli pyruvate oxidase, a lipid-activated flavoprotein"**

(73) Proprietor: **ASAHI KASEI KOGYO KABUSHIKI KAISHA**
**2-6, Dojimahama 1-chome**
**Kita-ku**
**Osaka(JP)**

(72) Inventor: **Matsumura, Eiji**
**530-1, Yoka-machi**
**Nirayama-cho**
**Tagate-gun Shizuoka-ken(JP)**
Inventor: **Imamura, Shigeyuki**
**696, Mifuku**
**Ohito-cho**
**Tagata-gun Shizuoka-ken(JP)**
Inventor: **Sagai, Hitoshi**
**128-51, Naka**
**Mishima-shi**
**Shizuoka-ken(JP)**
Inventor: **Misaki, Hideo**
**774, Yoshida**
**Ohito-cho**
**Tagata-gun Shizuoka-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

**JOURNAL OF BACTERIOLOGY, vol. 160, no. 1, October 1984, pages 273-278, American Society for Microbiology; B. SEDEWITZ et al.: "Purification and biochemical characterization of pyruvate oxidase from Lactobacillus plantarum"**

**JOURNAL OF BACTERIOLOGY, vol. 160, no. 3, December 1984, pages 1088-1092, American Society for Microbiology; C. GRABAU et al.: "Molecular cloning of the gene (poxB) encoding the pyruvate oxidase of Escherichia coli, a lipid-activated enzyme"**

**BIOCHIMICA ET BIOPHYSICA ACTA, vol. 452, 1976, pages 13-29, Elsevier/North-Holland, NL; T.A. O'BRIEN et al.: "Preparation of Escherichia coli pyruvate oxidase utilizing a thiamine pyrophosphate affinity column"**

Inventor: **Nogata, Keiko**
**855, Yoka-machi**
**Nirayama-cho**
**Tagata-gun Shizuoka-ken(JP)**

(74) Representative: **Woods, Geoffrey Corlett et al**
**J.A. KEMP & CO.**
**14 South Square**
**Gray's Inn**
**London WC1R 5EU (GB)**

## Description

This invention relates to a pyruvate oxidase which is substantially free of ATP-ase and lactate oxidase.

Pyruvate oxidase is an enzyme which catalyzes the reaction of pyruvic acid, phosphate and oxygen to form acetylphosphate, carbon dioxide and hydrogen peroxide and has heretofore been derived from a strain of Lactobacillus delbruckii (Williams, F.R. & Hager, L.P. (1966) Arch. Biochem. Biophys., 116: 168-176), Pediococcus, Streptococcus or Aerococcus vilidans JP-A-58-40465.

Pyruvate oxidase is an oxidase on the substrate pyruvic acid, and hence it can be used for quantitative measurement of pyruvate in serum, further this enzyme can be used for the quantitative analysis of substrate which is related to various enzymes on pyruvate generating system such as glutamate - oxaloacetate transaminase, glutamate - pyruvate transaminase, lactate dehydrogenase or neuraminidase - N-acetylneuraminic acid aldorase, and the measurement of the enzyme activity of enzyme reaction systems which form pyruvates. So pyruvate oxidase is useful for a reagent in research and clinical diagnostics.

Quantitative determination of ADP using pyruvate oxidase has been known. Namely pyruvate is generated by an action of pyruvate kinase which forms ATP and pyruvate from ADP and phosphoenolpyruvate, and the thus generated pyruvate is oxidized by pyruvate oxidase from Pediococcus sp. B-0667, Streptococcus sp. B-0668 or Aerococcus vilidans IFO 12219 and IFO 12317 to form hydrogen peroxide which is measured. (Japan. Pat. Unexam. Publ. No. 59-15637). Also pyruvate oxidase from other origin has been knonw (ibid. No. 59-162877 and No. 59-159777).

Hitherto known pyruvate oxidase producing microorganims has disadvantages such as low productivity and contamination of other enzymes which cannot be removed, and hence high quality pyruvate oxidase can not be obtained.

Further detailed chemical structure of pyruvate oxidase, which catalyses a reaction from pyruvate, phosphate and oxygen to generate acetylphosphate, carbon dioxide and hydrogen peroxide, has not been reported.

A prior used pyruvate oxidase in an assay of ADP contains considerable amount of contaminant ATP-ase which resulted higher observation in blank. In an assay of ADP, phosphoenol pyruvate and pyruvate kinase is added in a specimen containing ADP to generate ATP and pyruvate, then the reaction mixture is reacted with pyruvate oxidase and generated $H_2O_2$ is measured. In this assay system contaminated ATP-ase catalyses a reaction;

$$ATP + H_2O \rightarrow ADP + Pi$$

A resulted ADP is overlapped to the original ADP in a specimen. Therefore contaminant ATP-ase should be removed as much as possible. Furthermore prior pyruvate oxidase is contaminated with lactacte oxidase which resulted an error for measuring generated or consumed $H_2O_2$ in an assay using pyruvate oxidase. Therefore contaminate lactate oxidase should be removed.

We have succeeded to isolate pyruvate oxidase gene and to determine primary structure, and established the high productivity by applying genetic engineering technique.

The resulted pyruvate oxidase of the present invention has substantially no contaminant of ATP-ase with content below 0.0005%. The said pyruvate oxidase can be used for an assay of ADP with no increased color blank in assay. Further it does not contain lactate oxidase.

An object of the present invention is to provide a polydeoxyribonucleic acid which comprises:

- being extraneous for the host, and
- having base sequence coding an amino acid sequence of a polypeptide consisting of pyruvate oxidase which catalyses a reaction from pyruvate, phosphate and oxygen to generate acetyl-phosphate, carbon dioxide and hydrogen perioxide or an amino acid sequence including a polypeptide consisting of said pyruvate oxidase.

Another object of the present invention is to provide a polypeptide consisting of pyruvate oxidase which catalyses a reaction from pyruvate, phosphate and oxygen to generate acetylphosphate, carbon dioxide and hydrogen peroxide or a polypeptide including said pyruvate oxidase having an amino acid sequence from N-terminal of the formula

A – Ser Asp Asn Lys Ile Asn Ile Gly Leu
10
Ala Val Met Lys Ile Leu Glu Ser Trp Gly
20
Ala Asp Thr Ile Tyr Gly Ile Pro Ser Gly
30
Thr Leu Ser Ser Leu Met Asp Ala Met Gly
40
Glu Glu Glu Asn Asn Val Lys Phe Leu Gln
50
Val Lys His Glu Glu Val Gly Ala Met Ala
60
Ala Val Met Gln Ser Lys Phe Gly Gly Asn

70
Leu Gly Val Thr Val Gly Ser Gly Gly Pro
80
Gly Ala Ser His Leu Ile Asn Gly Leu Tyr
90
Asp Ala Ala Met Asp Asn Ile Pro Val Val
100
Ala Ila Leu Gly Ser Arg Pro Gln Arg Glu
110
Leu Asn Met Asp Ala Phe Gln Glu Leu Asn
120
Gln Asn Pro Met Tyr Asp His Ile Ala Val
130
Tyr Asn Arg Arg Val Ala Tyr Ala Glu Gln
140
Leu Pro Lys Leu Val Asp Glu Ala Ala Arg
150
Met Ala Ile Ala Lys Arg Gly Val Ala Val
160
Leu Glu Val Pro Gly Asp Phe Ala Lys Val
170
Glu Ile Asp Asn Asp Gln Trp Tyr Ser Ser
180
Ala Asn Ser Leu Arg Lys Tyr Ala Pro Ile
190
Ala Pro Ala Ala Gln Asp Ile Asp Ala Ala
200
Val Glu Leu Leu Asn Asn Ser Lys Arg Pro
210
Val Ile Tyr Ala Gly Ile Gly Thr Met Gly
220
His Gly Pro Ala Val Gln Glu Leu Ala Arg
230
Lys Ile Lys Ala Pro Val Ile Thr Thr Gly
240
Lys Asn Phe Glu Thr Phe Glu Trp Asp Phe
250
Glu Ala Leu Thr Gly Ser Thr Tyr Arg Val
260
Gly Trp Lys Pro Ala Asn Glu Thr Ile Lue

270
Glu Ala Asp Thr Val Leu Phe Ala Gly Ser
280
Asn Phe Pro Phe Ser Glu Val Glu Gly Thr
290
Phe Arg Asn Val Asp Asn Phe Ile Gln Ile
300
Asp Ile Asp Pro Ala Met Leu Gly Lys Arg
310
His His Ala Asp Val Ala Ile Leu Gly Asp
320
Ala Gly Leu Ala Ile Asp Glu Ile Leu Asn
330
Lys Val Asp Ala Val Glu Glu Ser Ala Trp
340
Trp Thr Ala Asn Leu Lys Asn Ile Ala Asp
350
Trp Arg Glu Tyr Ile Asn Met Leu Glu Thr
360
Lys Glu Glu Gly Asp Leu Gln Phe Tyr Gln
370
Val Tyr Asn Ala Ile Asn Asn His Ala Asp
380
Glu Asp Ala Ile Tyr Ser Ile Asp Val Gly
390
Asn Ser Thr Gln Thr Ser Ile Arg His Leu
400
His Met Thr Pro Lys Asn Met Trp Arg Thr
410
Ser Pro Leu Phe Ala Thr Met Gly Ile Ala
420
Ile Pro Gly Gly Leu Gly Ala Lys Asn Thr
430
Tyr Pro Asp Arg Gln Val Trp Asn Ile Ile
440
Gly Asp Gly Ala Phe Ser Met Thr Tyr Pro
450
Asp Val Val Thr Asn Val Arg Tyr Asn Met
460
Pro Val Ile Asn Val Val Phe Ser Asn Thr

470
Glu Tyr Ala Phe Ile Lys Asn Lys Tyr Glu
480
Asp Thr Asn Lys Asn Leu Phe Gly Val Asp
490
Phe Thr Asp Val Asp Tyr Ala Lys Ile Ala
500
Glu Ala Gln Gly Ala Lys Gly Phe Thr Val
510
Ser Arg Ile Glu Asp Met Asp Arg Val Met
520
Ala Glu Ala Val Ala Ala Asn Lys Ala Gly
530
His Thr Val Val Ile Asp Cys Lys Ile Thr
540
Gln Asp Arg Pro Ile Pro Val Glu Thr Leu
550
Lys Leu Asp Ser Lys Leu Tyr Ser Glu Asp
560
Glu Ile Lys Ala Tyr Lys Glu Arg Tyr Glu
570
Ala Ala Asn Leu Val Pro Phe Arg Glu Tyr
580
Leu Glu Ala Glu Gly Leu Glu Ser Lys Tyr
590
Ile Lys − B (I)

wherein A is amino acid residue, hydrogen or acetyl and B is amino acid residue, -OH or -$NH_2$.

Further object of the present invention is to provide a process for production of pyruvate oxidase which comprises:

- transforming a recombinant DNA into a host microorganism, in which the said recombinant DNA is contracted by inserting a polydeoxyribonucleic acid consisting of gene of pyruvate oxidase which catalyses a reaction from pyruvate, phosphate and oxygen to generate acetylphosphate, carbon dioxide and hydrogen peroxide or a polydeoxyribonucleic acid including the said gene, to obtain a transformant,
- expressing a genetic information of said polydeoxyribonucleic acid by culturing said transformant, and
- isolating a polypeptide consisting of pyruvate oxidase which catalyses a reaction from pyruvate, phosphate and oxygen to generate acetylphosphate, carbon dioxide and hydrogen peroxide, or a polypeptide including pyruvate oxidase.

More further object of the present invention is to provide a composition for assay comprising water soluble pyruvate oxidase which catalyses a reaction from pyruvate, phosphate and oxygen to generate acetylphosphate, carbon dioxide and hydrogen peroxide, with ATPase content of at least below 0.0005% and substantially containing no lactate oxidase activity.

Still further object of the present invention is to provide an assay method which comprises, in an assay of pyruvate in a specimen which contains pyruvate or generates pyruvate and at least contains lactate, treating said specimen with water soluble pyruvate oxidase which catalyses a reaction from pyruvate, phosphate and oxygen to generate acetylphosphate, carbon dioxide and hydrogen peroxide, with ATPase content of at least below 0.0005% and substantially containing no lactate oxidase activity, and measuring a consumed or generated composition.

Still more further object of the present invention is to provide an assay method of ADP which comprises at least including the following processes:

(a) a process generating ATP and pyruvate from ADP and phosphoenol pyruvate by an action of transphosphorylation of pyruvate kinase,

(b) a process forming detectable changes by generating acetylphosphate, carbon dioxide and hydrogen perioxide by an action of pyruvate oxidase on a substrate pyruvate using a composition of an effective ingredient consisting of water soluble pyruvate oxidase which catalyses a reaction from pyruvate, phosphate and oxygen to generate acetylphosphate, carbon dioxide and hydrogen peroxide, with ATPase content of at least below 0.0005%, and

(c) a process detecting the said detectable changes by acting enzyme and reagent.

Still another further object of the present invention is to provide an assay method of ATP which comprises at least including the following processes:

(a) a process generating ADP and phosphate compound from ATP and non-phosphate compound by an action of transphosphorylation of kinase,

(b) a process generating ATP and pyruvate form generated ADP and phosphenol pyruvate by an action of transphosphorylation of pyruvate kinase,

(c) a process forming detectable changes by generating acetylphosphate, carbon dioxide and hydrogen peroxide by an action of pyruvate oxidase on a substrate pyruvate using a composition of an effective ingredient consisting of water soluble pyruvate oxidase which catalyses a reaction from pyruvate, phosphate and oxygen to generate acetylphosphate, carbon dioxide andhydrogen peroxide, with ATPase content of at least below 0.0005%, and

(d) a process detecting the said detectable changes by acting enzyme and reagent.

Still furthermore object of the present invention is to provide an assay method of non-phosphate compound which comprises at least including the following processes:

(a) a process generating ADP and phosphate compound from ATP and non-phosphate compound by an action of transphosphorylation of kinase,

(b) a process generating ATP and pyruvate form generated ADP and phosphoenol pyruvate by an action of transphosphorylation of pyruvate kinase,

(c) a process forming detectable changes by generating acetylphosphate, carbon dioxide and hydrogen peroxide by an action of pyruvate oxidase on a substrate pyruvate using a composition of an effective ingredient consisting of water soluble pyruvate oxidase which catalyses a reaction from pyruvate, phosphate and oxygen to generate acetylphosphate, carbon dioxide and hydrogen peroxide, with ATPase content of at least below 0.0005%, and

(d) a process detecting the said detectable changes by acting enzyme and reagent.

In a polypeptide (I) amino acid residue A is one or more of amino acid residue, preferably hydrogen, Met or signal peptide. Example of B is acidamide or amino acid residue of more than one.

An example of a polydeoxyribonucleic acid of a gene consisting of pyruvate oxidase which catalyses a reaction from pyruvate, phosphate and oxygen to generate acetyl-phosphate, carbon dioxide and hydrogen peroxide or a polydeoxyribonucleic acid including said gene is at least a polydeoxyribonucleic acid containing gene of pyruvate oxidase which catalyses a reaction from pyruvate, phosphate and oxygen to generate acetylphosphate, carbon dioxide and hydrogen peroxide, and is a polydeoxyribonucleic acid consisting of a base sequence coding an amino acid sequence of a polypeptide of pyruvate oxidase having a structure from N-terminal of the formula

Ser Asp Asn Lys Ile Asn Ile Gly Leu
10
Ala Val Met Lys Ile Leu Glu Ser Trp Gly
20
Ala Asp Thr Ile Tyr Gly Ile Pro Ser Gly
30
Thr Leu Ser Ser Leu Met Asp Ala Met Gly
40
Glu Glu Glu Asn Asn Val Lys Phe Leu Gln
50
Val Lys His Glu Glu Val Gly Ala Met Ala
60
Ala Val Met Gln Ser Lys Phe Gly Gly Asn

70
Leu Gly Val Thr Val Gly Ser Gly Gly Pro
80
Gly Ala Ser His Leu Ile Asn Gly Leu Tyr
90
Asp Ala Ala Met Asp Asn Ile Pro Val Val
100
Ala Ila Leu Gly Ser Arg Pro Gln Arg Glu
110
Leu Asn Met Asp Ala Phe Gln Glu Leu Asn
120
Gln Asn Pro Met Tyr Asp His Ile Ala Val
130
Tyr Asn Arg Arg Val Ala Tyr Ala Glu Gln
140
Leu Pro Lys Leu Val Asp Glu Ala Ala Arg
150
Met Ala Ile Ala Lys Arg Gly Val Ala Val
160
Leu Glu Val Pro Gly Asp Phe Ala Lys Val
170
Glu Ile Asp Asn Asp Gln Trp Tyr Ser Ser
180
Ala Asn Ser Leu Arg Lys Tyr Ala Pro Ile
190
Ala Pro Ala Ala Gln Asp Ile Asp Ala Ala
200
Val Glu Leu Leu Asn Asn Ser Lys Arg Pro
210
Val Ile Tyr Ala Gly Ile Gly Thr Met Gly
220
His Gly Pro Ala Val Gln Glu Leu Ala Arg
230
Lys Ile Lys Ala Pro Val Ile Thr Thr Gly
240
Lys Asn Phe Glu Thr Phe Glu Trp Asp Phe
250
Glu Ala Leu Thr Gly Ser Thr Tyr Arg Val
260
Gly Trp Lys Pro Ala Asn Glu Thr Ile Lue

270
Glu Ala Asp Thr Val Leu Phe Ala Gly Ser
280
Asn Phe Pro Phe Ser Glu Val Glu Gly Thr
290
Phe Arg Asn Val Asp Asn Phe Ile Gln Ile
300
Asp Ile Asp Pro Ala Met Leu Gly Lys Arg
310
His His Ala Asp Val Ala Ile Leu Gly Asp
320
Ala Gly Leu Ala Ile Asp Glu Ile Leu Asn
330
Lys Val Asp Ala Val Glu Glu Ser Ala Trp
340
Trp Thr Ala Asn Leu Lys Asn Ile Ala Asp
350
Trp Arg Glu Tyr Ile Asn Met Leu Glu Thr
360
Lys Glu Glu Gly Asp Leu Gln Phe Tyr Gln
370
Val Tyr Asn Ala Ile Asn Asn His Ala Asp
380
Glu Asp Ala Ile Tyr Ser Ile Asp Val Gly
390
Asn Ser Thr Gln Thr Ser Ile Arg His Leu
400
His Met Thr Pro Lys Asn Met Trp Arg Thr
410
Ser Pro Leu Phe Ala Thr Met Gly Ile Ala
420
Ile Pro Gly Gly Leu Gly Ala Lys Asn Thr
430
Tyr Pro Asp Arg Gln Val Trp Asn Ile Ile
440
Gly Asp Gly Ala Phe Ser Met Thr Tyr Pro
450
Asp Val Val Thr Asn Val Arg Tyr Asn Met
460
Pro Val Ile Asn Val Val Phe Ser Asn Thr

470
Glu Tyr Ala Phe Ile Lys Asn Lys Tyr Glu
480
Asp Thr Asn Lys Asn Leu Phe Gly Val Asp
490
Phe Thr Asp Val Asp Tyr Ala Lys Ile Ala
500
Glu Ala Gln Gly Ala Lys Gly Phe Thr Val
510
Ser Arg Ile Glu Asp Met Asp Arg Val Met
520
Ala Glu Ala Val Ala Ala Asn Lys Ala Gly
530
His Thr Val Val Ile Asp Cys Lys Ile Thr
540
Gln Asp Arg Pro Ile Pro Val Glu Thr Leu
550
Lys Leu Asp Ser Lys Leu Tyr Ser Glu Asp
560
Glu Ile Lys Ala Tyr Lys Glu Arg Tyr Glu
570
Ala Ala Asn Leu Val Pro Phe Arg Glu Tyr
580
Leu Glu Ala Glu Gly Leu Glu Ser Lys Tyr
590
Ile Lys (II)

In an amino acid sequence of polypeptide (II) of pyruvate oxidase, any polydeoxyribonucleic acid comprising any codon corresponding to each amino acid can be mentioned, and preferably it can be a polydeoxyribonucleic acid having more than one codon except nonsense codon at 5'-terminal and/or more than one codon at 3'-terminal. For example a polydeoxyribonucleic acid having base sequence from 5' -terminal of the formula

X — TCA GAT AAC AAA ATT AAC ATC GGT TTA
30
GCA GTT ATG AAG ATT TTA GAA TCT TGG GGA
60
GCA GAT ACT ATT TAT GGT ATT CCT TCA GGT
90
ACT TTA AGC TCA TTA ATG GAT GCT ATG GGT
120
GAA GAA GAA AAC AAC GTC AAA TTC CTA CAA
150
GTG AAA CAC GAA GAA GTA GGT GCA ATG GCT
180
GCT GTA ATG CAA AGC AAA TTC GGC GGT AAC
210
TTA GGT GTT ACT GTA GGT TCT GGT GGA CCA
240
GGT GCA TCT CAC TTG ATC AAC GGT TTA TAC
270
GAT GCA GCA ATG GAT AAC ATT CCA GTA GTT
300
GCG ATC TTA GGT TCT CGT CCA CAA CGC GAA
330
TTA AAC ATG GAC GCT TTC CAA GAA TTA AAC

360
CAG AAC CCA ATG TAC GAC CAT ATT GCA GTT
390
TAC AAC CGT CGT GTA GCT TAT GCT GAG CAA
420
TTA CCA AAA TTA GTT GAC GAA GCA GCT CGT
450
ATG GCT ATC GCT AAA CGC GGT GTA GCA GTT
480
CTA GAA GTA CCT GGT GAT TTT GCT AAA GTT
510
GAA ATC GAC AAC GAC CAA TGG TAT TCA TCT
540
GCA AAC AGC TTA CGT AAA TAT GCA CCA ATC
570
GCT CCA GCA GCA CAA GAT ATC GAC GCA GCA
600
GTT GAA TTA TTA AAC AAC TCT AAA CGT CCA
630
GTT ATC TAC GCT GGT ATT GGT ACT ATG GGC
660
CAC GGT CCT GCA GTT CAA GAA TTA GCT CGT
690
AAA ATC AAA GCG CCA GTT ATC ACT ACT GGT
720
AAA AAC TTT GAA ACT TTC GAG TGG GAT TTC
750
GAA GCG TTA ACA GGT TCT ACT TAT CGT GTA
780
GGT TGG AAA CCA GCT AAC GAA ACA ATT TTA
810
GAA GCT GAC ACA GTA TTA TTT GCT GGT TCA
840
AAC TTC CCA TTC TCA GAG GTT GAA GGT ACT
870
TTC CGT AAC GTG GAT AAC TTC ATC CAA ATC
900
GAT ATC GAC CCA GCT ATG TTA GGT AAA CGT
930
CAC CAC GCT GAT GTT GCT ATC TTA GGT GAT

960
GCT GGT CTA GCA ATC GAC GAA ATC TTA AAC
990
AAA GTA GAT GCT GTT GAA GAG TCA GCA TGG
1020
TGG ACA GCT AAC TTG AAA AAC ATC GCT AAC
1050
TGG CGT GAA TAC ATC AAC ATG TTA GAA ACT
1080
AAA GAA GAA GGC GAC TTG CAA TTC TAC CAA
1110
GTG TAC AAT GCA ATC AAC AAC CAC GCC GAC
1140
GAA GAT GCA ATC TAC TCT ATT GAT GTT GGT
1170
AAC TCA ACT CAA ACT TCT ATC CGT CAT TTA
1200
CAT ATG ACA CCT AAA AAC ATG TGG AGA ACT
1230
TCT CCA TTA TTC GCG ACA ATG GGT ATC GCT
1260
ATC CCT GGT GGT TTA GGT GCT AAA AAC ACT
1290
TAC CCA GAT CGT CAA GTT TGG AAC ATC ATC
1320
GGT GAT GGT GCT TTC TCT ATG ACT TAC CCA
1350
GAT GTA GTA ACT AAC GTA CGT TAC AAC ATG
1380
CCT GTA ATC AAC GTT GTA TTC TCT AAC ACT
1410
GAA TAT GCC TTC ATC AAA AAC AAA TAT GAA
1440
GAC ACT AAC AAA AAC TTA TTC GGT GTA GAC
1470
TTT ACA GAT GTT GAT TAC GCT AAA ATC GCT
1500
GAA GCA CAA GGT GCT AAA GGA TTT ACT GTA
1530
AGC CGT ATC GAA GAT ATG GAC CGT GTA ATG

1560
GCT GAA GCT GTT GCA GCT AAT AAA GCA GGT
1590
CAC ACT GTC GTT ATC GAC TGT AAG ATT ACT
1620
CAA GAT CGT CCA ATC CCT GTA GAA ACA TTG
1650
AAA TTA GAT TCA AAA CTT TAC AGC GAA GAC
1680
GAA ATC AAA GCT TAC AAA GAA CGC TAC GAA
1710
GCT GCT AAC TTA GTA CCA TTC AGA GAG TAC
1740
TTA GAA GCT GAA GGC TTA GAA TCT AAA TAC
1770
ATC AAA − Y (III)

wherein X is a codon excepting TAA, TAG and TGA or hydrogen, and Y is codon or hydrogen can be mentioned.

In a base sequence (III), codon X can be a codon coding amino acid, and it can be a codon more than one which codes amino acid at 5'-terminal, and is preferably ATG or polydeoxyrebonucleic acid corresponding to signal peptide.

A codon Y can be a termination codon or amino acid coding codon, further it may have more than one codon which codes amino acid at 3'-terminal and is preferably to have a termination codon at 3'-terminal of plural codons.

A polydeoxyribonucleic acid comprising pyruvate oxidase gene, a polydeoxyribonucleic acid consisting of a gene having base sequence which codes amino acid sequence (II) or a polydeoxyribonucleic acid (III) can be obtained, for example, by isolating DNA from pyruvate oxidase producing microorganisms which is a donor of pyruvate oxidase gene, splitting the DNA by sonication or digstion with restriction enzyme, ligating the DNA with linear expression vector at the blunt end or cohesive end by DNA ligase, transferring the thus obtained recombinant DNA vector into host microorganism, obtaining microorganisms carrying the recombinant plasmid by selecting for the vector markers and activity of pyruvate oxidase, culturing the said microorganims, isolating and purifying the said recombinant DNA vector from the cultured cells, and isolating the polydeoxyribo-nucleic acid of pyruvate oxidase gene from the said recombinant DNA vector.

A microorganism which is a donor of pyruvate oxidase gene can be a pyruvate oxidase producing microorganism, for example, Lactobacillaceae and Streptococcaceae, such as Lactobacillus delbrucki - [Japan. Pat. Unexam. Publ. No. 54-126791, No. 59-159777, No. 59-162877 and Arch. Biochem. Biophys., 116: 168 - 176 (1966)]. Pediococcus sp., Streptococcus sp., Aerococcus viridans, Lactobacillus salivarius, Leuconostoc mesenteroides.

Also transformed microorganisms which is given pyruvate oxidase producing activity by means of recombinant DNA technology, can be used as a donor of pyruvate oxidase gene.

DNA can be obtained from gene donor microorganisms as follows. Microorganisms exemplified hereinabove are cultured for 1 - 3 days aerobically in a liquid medium. Cultured cells are collected by centrifugation and harvested cells are lysed to obtain lysate cells containing pyruvate oxidase gene. Bacteriolysis can be made by treatment of cell wall lytic enzyme such a slysozyme or β-glucanase optionally treated with other enzymes such as protease or surface active agent such as sodium lauryl sulfate and other physical destruction means such as freeze-thawing or French press.

Isolation and purification of DNA from lytic cells can be made by combination of conventional means such as deproteination by phenol treatment, treatment with protease and/or, ribonuclease, alcohol precipitation or centrifugation.

Splitting of isolated or purified microbial DNA can be made by sonic treatment or restriction enzyme treatment, preferably restriction enzyme treatment for easy ligation, especially using enzyme acting on

specific nucleotide sequence, for example type II restriction enzyme such as EcoRI, HindIII or BamHI.

Preferable vectors are plasmids constructed for recombinant DNA technology from autonomically grown phage or plasmid in host cells.

Examples of phage are λgt • λC and λgt • λB for host cell of Escherichia coli.

Examples of plasmid are pBR322, pBR325, pACYC184, pUC12 pUC13, pUC18 or pUC19 for E. coli , pUB110 or pC194 for Bacillus subtillis , and shuttle vector for E. coli and Saccharomyces cerevisiae. It is preferable to obtain vector fragment by cleaving the vector hereinabove with the same restriction enzyme used in cleavage of microbial DNA containing pyruvate oxidase gene hereinbefore.

Ligating method of microbial DNA fragment and vector fragment can be a conventional method using known DNA ligase. For example after annealing a cohesive end of microbial DNA fragment a nd cohesive end of vector fragment, a recombinant DNA of microbial DNA fragment and vector fragment can be constructed by an action of preferable DNA ligase. If necessary, after annealing, DNA is transforred into host microorganisms and a recombinant DNA can be prepared by means of in vivo DNA ligase.

Examples of host microorganism are preferably a microorganism in which recombinant DNA can be replicatable in stable and autonomically and transforred extraneous DNA can be expressed, for example Escherichia coli DH1, E. coli HB101, E. coli W3110 and E. coli C600.

As for a method of transfer of recombinant DNA into a host microorganism, for example in case that the host cell is E. coli, a recombinant DNA can be transferrd in the presence of calcium ion, and in case that the host cell is Bacillus, a competent cell method or protoplast method can be applied, further microinjection method may also be applied. The thus obtained and cultured transformant can produce large amount of pyruvate a oxidase stably in a culture of nutrient medium.

Insertion of recombinant DNA can be selected by isolating the microorganisms which express drug resistant markers in the said vector and pyruvate oxidase production. For example micro -organisms which can grow in a selection medium for drug resistance and have activity of pyruvate oxidase production, is preferably selected.

Selected recombinant DNA consisting of pyruvate oxidase gene is isolated from transformed cells and can easily transform the other host cells. Also pyruvate oxidase gene consisting of DNA is digested by restriction enzyme to obtain DNA fragment of pyruvate oxidase gene, which is ligated with vector fragment obtained by the same way, and the ligated DNA can easily be transferred into the host cells.

DNA coding pyruvate oxidase mutein which shows substantial pyruvate oxidase activity, is an artificial mutant gene derived from pyruvate oxidase gene of the present invention by means of genetic engineering technique and this mutant gene which is prepared by the methods hereinbefore is inserted into a vector to construct recombinant DNA, then pyruvate oxidase protein can be produced.

A base sequence of the thus obtained pyruvate oxidase gene is determined by dideoxy method (Science, 214: 1205 - 1210, 1981) and amino acid sequence of pyruvate oxidase is predicted by the base sequence.

A partial amino acid sequence of N-terminal of pyruvate oxidase peptide is determined by the following method. Pyruvate oxidase producing microorganisms which is a donor of pyruvate oxidase gene is cultured in a nutrient medium to accumulate pyruvate oxidase endogeneously. The cultured cells are collected by filtration or centrifugal means, disrupted by mechanically means or enzymatically with lysozyme, solubilized by adding EDTA and/or surface active agent and isolated the enzyme solution. Aqueous solution of pyruvate oxidase is treated by ammonium sulfate fractionation, gel-filtration, adsorption chromatography or ion-exchange chromatography with or without concentration to obtain highly purified pyruvate oxidase. A partial sequence of amino acid of N-terminal of pyruvate oxidase is determined by the liquid phase protein sequencer (Beckman System 890 ME) and confirmed the identity as compared with that of pyruvate oxidase obtained by genetic manipulation.

Cultivation condition of the transformant is determined by considering nutrient physiological properties of cells, and is usually made by conventional liquid culture and submerged aeration culture is preferable for industrial production.

A conventional medium for culturing microorganisms can preferably be used. For the carbon sources, assimilable carbon sources such as glucose, sucrose, lactose, maltose, fructose, molasses, pyruvic acid or the like can preferably be used. Assimilable nitrogen sources such as peptone, meat extract, yeast extract, casein hydrolyzate or the like can be used. Various inorganic salts such as phosphates, carbonates, sulfates, salts of magnesium, calcium, potassium, divalent iron, manganese or zinc or specific amino acids and vitamins can be used.

The culturing temperature can be selected within the range for growth of microbial cells and production of pyruvate oxidase, and is preferably 20-42 °C for E. coli. The culturing time can be altered depending on conditions and is terminated when the pyruvate oxidase production is substantially complete, and is usually

12-48 hours.

The pH of the medium can be altered depending on conditions for growing cells and producing pyruvate oxidase, and is usually pH 6.0-8.0.

To separate pyruvate oxidase from the culture, the cultured mass is filtered or centrifuged to collect the cells, which are disrupted by treatment with mechanical means or enzymes such as lysozyme or in case that enzyme is exogeneously existed in a medium, cultured mass is filtered or centrifuged to separate culture filtrate. Further if necessary pyruvate oxidase is solubilized by adding EDTA and a surfactant to extract the enzyme. The thus-obtained solution of pyruvate oxidase is treated with or without concentration, and thereafter the enzyme is precipitated by salting out with the addition of a soluble salt such as ammonium sulfate or sodium chloride. Low molecular weight impurities are removed by dialysis. Furthermore impurities in the solution of pyruvate oxidase are preferably removed by adsorption chromatography, ion-exchange chromatography or gel filtration. The enzyme solution thus obtained is treated by vacuum concentration and lyophilization to produce powdered pyruvate oxidase.

Abbreviations of amino acid, peptide, nucleic acid, nucleic acid related substance and others are as follows.

All the amino acid shows L-form.

DNA: deoxyribonucleic acid
RNA: ribonucleic acid
A: adenine
T: thymine
G: guanine
C: cytocine
Ala: alanine
Arg: arginine
Asn: asparagine
Asp: aspartate
Cys: cysteine
Gln: glutamine
Glu: glutamate
Gly: glycine
His: histidine
Ile: isoleucine
Leu: leucine
Lys; lysine
Met: methionine
Phe: phenylalanine
Pro: proline
Ser: serine
Thr: threonine
Trp: tryptophane
Tyr: tyrosine
Val: valine

Pyruvate oxidase produced by the present invention has the following physico-chemical properties,

(1) Enzyme action:

The enzyme catalyzes the oxidative reaction of pyruvic acid, inorganic phosphate and oxygen to form acetylphosphate, carbon dioxide and hydrogen peroxide:

$$CH_3COCOOH + HOPO_3^{-} + O_2 \rightarrow CH_3COOPO_3^{-} + CO_2 + H_2O_2$$

(2) Substate specificity: Specific for pyruvate.

No action on α-ketoglutarate, oxaloacetate, lactate, acetate and alanine.

(3) Isoelectric point: pH 4

(4) Molecular weight: 150,000 - 155,000 (gel-filtration method)

(5) Optimum pH : 6.5 - 7.5

(6) pH-stability: 5.5 - 7

(7) Lactate oxidase activity:

Substantially no lactate oxidase activity is observed. Activity of lactate oxidase is measured according to Japan. Pat. Unexam. Pat. Publ. No. 55-76.

(8) ATP-ase activity:
Contaminant ATP-ase activity is observed less than 0.0005%.
(9) Nature: soluble in water.
(10) Amino acid sequence: shown as formula (I).

The assay method of pyruvate oxidase of the present invention uses a reaction mixture as follows:

| | |
|---|---|
| 0.5 M potassium pyruvate | 0.1 ml |
| 0.5 M phosphate buffer (pH 7.0) | 0.2 ml |
| 0.2% 4-aminoantipyrin | 0.1 ml |
| 0.2% N,N-dimethylaniline | 0.2 ml |
| 10 mM $MgCl_2$ | 50 µl |
| 10 mM thiaminepyrophosphate | 20 µl |
| peroxidase (45 U/ml) | 0.1 ml |
| 1 mM FAD | 10 µl |
| distilled water | 0.22 ml |

The above reaction mixture (1.0 ml) is pre-incubated at 37°C for 3 minutes. To this solution is added the enzyme solution (20 µl) and incubated at 37°C for 10 minutes. 0.1 M citrate buffer (pH 6.0, 2 ml) containing 0.1 M EDTA is added to stop the reaction. The violet color formed is measured by colorimetric method at 565 nm.

A unit (1 unit, 1 U) of enzyme activity is defined as the activity which generates 1 µmole of hydrogen peroxide per minute.

Fundamental reactions of assay method using pyruvate oxidase are illustrated as follows.

I. Pyruvate generating system

In the assay, the following reaction can at least be included and various combination of known prior reactions therefor can be used.

① a reaction of glutamate-pyruvate-transaminase:
alanine + α-ketoglutarate → pyruvate + glutamate
② a reaction of glutamate-oxaloacetate-transaminase and oxaloacetate decarboxylase:
aspatate + α-ketoglutarate → oxaloacetate + glutamate oxaloacetate → pyruvate + $CO_2$
③ a reaction of pyruvate kinase:
ADP + phosphoenolpyruvate → ATP + pyruvate
④ a reaction of N-acetyleneulaminate aldolase:
N-acetyleneulaminate + $H_2O$ → N-acetylmanosamine + pyruvate
⑤ a reaction of lactate dehydrogenase:
lactate + NAD → pyruvate + reduced NAD

Preferable combination for the above reaction is, for example, ammonium generated from urea by an action of urease, ammonium generated from creatinine by an action of creatinine diaminase, or ammonium liberated from the other reaction system, is reacted with glutamate and ATP in the presence of glutamine synthetase to form ADP, glutamine and inorganic phosphate, and the thus generated ADP is combined with ADP assay system using pyruvate kinase and phosphoenolpyruvate hereinabove.

II Assay of ADP :

(a)

$$\text{ADP} + \text{phosphoenolpyruvate} \xrightarrow{\text{pyruvate kinase}} \text{ATP} + \text{pyruvate}$$

(b)

$$pyruvate + Pi + O_2 \xrightarrow{\text{pyruvate oxidase}} acetyphosphate + CO_2 + H_2O_2$$

(c) measuring consumed $O_2$ or generated $H_2O_2$ or $CO_2$

Various assay can be made by combining prior reaction systems to the above processes.

III Assay of ATP:

(a)

$$ATP + \text{non-phosphte compound} \xrightarrow{\text{kinase}} ADP + \text{phosphate compound}$$

(b) ADP + phosphoenolpyruvate → ATP + pyruvate

(c)

$$pyruvate + Pi + O_2 \xrightarrow{\text{pyruvate oxidase}} acetylphosphate + CO_2 + H_2O_2$$

(d) measuring consumed $O_2$ or generated $H_2O_2$ or $CO_2$

IV Assay of non-phosphate compound:

The same as of the above III.

Examples of ADP generating or consuming enzyme reaction system are illustrated as follows.

ADP generating reaction:

① a reaction of hexokinase;
ATP + D-hexose → ADP + D-hexose-6-phosphate
② a reaction of glucokinase;
ATP + D-glucose → ADP + D-glucose-6-phosphate
③ a reaction of amylase (with maltase);
glucose polymer (soluble starch, amylose or other oligosaccharide or derivatives thereof) + $nH_2O$ → D-glucose + maltose, and
maltose + $2H_2O$ → 2 D-glucose
ATP + D-glucose → ADP + D-glucose-6-phosphate
④ a reaction of adenosine kinase;
ATP + adenosine → ADP + AMP
⑤ a reaction of thymidine kinase;
ATP + thymidine → ADP + thymidine-5′-phosphate
⑥ a reaction of NAD kinase;
ATP + $NAD^+$ → ADP + $NADP^+$
⑦ a reaction of enzyme action from NADH + $H^+$ to generate $NAD^+$
NADH + $H^+$ + substrate A (oxydized form) → $NAD^+$ + $H_2A$ and ATP + $NAD^+$ → ADP + $NADP^+$

⑧ a reaction of riboflavine kinase;
ATP + riboflavin → ADP + flavin -5′-phosphate
⑨ a reaction of glycerol kinase;
ATP + glycerol → ADP + glycerol-3-phosphate
⑩ a reaction of triglyceride assay (with lipase);
triglyceride + $3H_2O$ → glycerol + 3 fatty acid, and
ATP + glycerol → ADP + glycerol-3-phosphate
⑪ a reaction of lipase assay;
di- or triglyceride + $nH_2O$ → glycerol + n fatty acid, and
ATP + glycerol → ADP + glycerol-3-phosphate
⑫ a reaction of choline kinase;
ATP + choline → ADP + choline phosphate
⑬ a reaction of choline esterase assay ;
choline ester (fatty acid ester or aryl ester; RCOO) + $H_2O$ → choline + $RCOO^-$ and
ATP + choline → ADP + choline phosphate
⑭ a reaction of phospholipid (lecithin) assay (with phospholipase D);
lecithin + $H_2O$ → phosphatidate + choline ATP + choline → ADP + choline phosphate
⑮ a reaction of protein kinase assay;
ATP + protein → ADP + phospho-protein
⑯ an assay of creatine kinase or creatine;
ATP + creatine → ADP + creatine phosphate

ATP generating reaction (ADP-consuming reaction):

① a reaction of carbamate kinase assay, ADP or carbamoyl-phosphate assay;
ADP + carbamoyl phosphate → $NH_3$ + ATP + $CO_2$
② an assay of phosphoglycerate kinase activity or ADP;
ADP + D-1,3-bisphosphoglycerate → ATP + 3-glycerol-D-glycetrate
③ a reaction of formate kinase assay, ADP or formyl-phosphate;
ADP + formyl phosphate → ATP + formate
④ a reaction of creatine kinase assay, ADP or creatine-phosphate assau;
ADP + creatine phosphate → ATP + creatine
⑤ an assay of ammonia kinase activity or ADP;
ADP + phosphoramide → ATP + $NH_4^+$
⑥ an assay of myokinase activity or ADP;
2ADP → ATP + AMP

An assay method of generated ADP or consumed ADP is illustrated in Japanese Patent Appln. No. 53-86350 "assay kit and method using pyruvate oxidase".

In order to activate the pyruvate oxidase reaction system, FAD, thiaminepyrophosphate and phosphate are added. Further for activation of the enzyme, an ion-liberating salt which liberates calcium ions, cobalt ions, magnesium ions or manganese ions, in the form of chloride is preferably added thereto. An indicator such as a coloring indicator or fluorescent indicator for hydrogen peroxide can preferably be used.

The amount and ratio of components in the enzyme reaction system can be selected for substantial enzyme reaction and will be varied according to the amount of pyruvate, temperature and time of enzyme reaction. For example, 1-200 U of pyruvate oxidase can preferably be used.

Pyruvate oxidase can be in a microcapsulated form or in an immobilized form of covalent linkage with an organic or inorganic carrier or adsorbed on a carrier.

Further 0.1 - 20 nmoles of FAD, 0.05-0.5 $\mu$mole of thiaminepyrophosphate, 1-10 $\mu$moles of inorganic phosphate and 0.05-0.1 $\mu$moles of ion liberating salt per test can preferably be used.

The molar ratio of indicator for hydrogen peroxide is at least an equimolar or excess amount of generated hydrogen peroxide. In the case of the peroxidase, 0.5-20 U per test is preferably used. These components of the enzymatic reaction mixture are preferably used by dissolving in the buffer of suitably adjusted pH.

The thus-prepared enzymatic reaction system is used for the analysis of pyruvic acid, ADP, ATP or related non-phosphate compound.

Assay is performed by incubation with the sample and a reagent mixture. The reagent mixture is preferably a kit of necessary reagents. For assaying, consumed component or generated component is measured. Measuring the amount of oxygen consumption by dissolved oxygen meter is preferable as an

assay method. In this case no indicator for hydrogen peroxide is necessary. As for the assaying of a generated component, measurement of the amount of hydrogen peroxide is preferable, for example by a hydrogen peroxide electrode meter such as YSI-oxidase meter or by colorimetric or fluorometric assay with an indicator for hydrogen peroxide. The assay can be performed preferably for 10-60 minutes and at 20-40 °C, preferably at 35-37 °C.

The indicator for hydrogen peroxide is a combination of one or more chromogen or fluorescent, which is effected by coupling with hydrogen peroxide. Examples of such indicators are combinations of tetravalent titanium compounds and xylenol orange which couples with hydrogen peroxide to form a stable red color, or a combination of phenol or N,N-dimethylaniline or homovanillic acid, 4-aminoantipyrine and peroxidase for measuring color or fluorescence. 4-aminoantipyrine can be replaced by 4-aminophenazone. A combination of 2,6-dichlorophenol indophenol and peroxidase and of guaiacol and peroxidase can also be used. The indicator can be previously prepared as a solution.

The amount of pyruvic acid can be measured by calculation from corresponding standard curves of consumed oxygen or generated hydrogen peroxide.

Colorimetric or fluorometric assy is performed by measuring the absorption at a suitable wave length such as 565 nm.

Phosphate as a consumed component or acetylphosphate as a generated component can also be assayed by any conventional method.

Pyruvate oxidase of the present invention does not contaminated with lactate oxidase and content of ATP-ase is less than 0.0005%, and hence is advantageous as compared with prior known pyruvate oxidase which contains lactate oxidase and 0.005% of ATP-ase.

ATP-ase assay method and coloring reagent for ADP assay are illustrated as follows.

1. ATP-ase assay:

Reagent 1 (1.0 ml)(containing 40 U pyruvate oxidase) is preincubated at 37 °C for 1 hour. ADP reagent 2 (1.0 ml) is added thereto, and incubated at 37 °C for 15 min., further added 10% SDS (sodium dodecyl sulfate) (1.0 ml) to dissolve turbidity, and measured at 550 nm.

Reagent 1:

| | |
|---|---|
| 0.2 M PIPES buffer (pH 7) | 0.2 ml |
| 0.1 M $MgCl_2$ | 0.08 ml |
| 0.2 M ATP (Sigma) | 0.01 ml |
| pyruvate oxidase (40 u) | 0.1 ml |
| $H_2O$ | 0.61 ml |
| | 1.0 ml |

Reagent 2:

| | |
|---|---|
| 0.2 M Tris-HCl buffer (pH 7.5) | 0.1 ml |
| 10 mM phosphoenolpyruvate (Boehringer) | 0.1 ml |
| pyruvate oxidase (500 u/ml, Toyo Jozp) | 0.01 ml |
| 10 mM thiamine pyrophosphate (Wako) | 0.02 ml |
| 1 mM FAD (Sigma) | 0.01 ml |
| 0.1 M $MgCl_2$ | 0.1 ml |
| 0.3% 4-aminoantipyrine | 0.1 ml |
| 0.3% TOOS | 0.1 ml |
| peroxidase (50 u/ml, Sigma) | 0.1 ml |
| 1 M $KH_2PO_4$ (pH 7.5) | 0.05 ml |
| $H_2O$ | 0.305 ml |
| | 1.0 ml |

· ATP-ase activity (u/ml)

$$= \frac{\Delta A_{550\ nm}}{18} \times \frac{3.0}{1.0} \times \frac{1}{60}$$

$\Delta A_{550\ nm}$ : In the reagent 1, ATP is deleted. Incubation was carried as same way and absorbancy was deducted.
18 : molecular absorbancy coefficient
0.1 : enzyme solution (1 ml)
3.0 : total reaction volume (ml)
60 : reaction time (min.)

- ATP-ase contamination ratio:

$$\text{ATP-ase (u/ml)} \times \frac{1}{400}$$

400 : pyruvate oxidase activity (400 u/ml).

2. Comparison of coloring reagent for ADP assay:

| | Concentration | |
|---|---|---|
| | Optimum range | Conventional range |
| Tris-HCl (pH 7.5)* | 50 mM | 20-200 mM |
| phosphoenol pyruvate (Boehringer) | 1.0 mM | 0.2-5 mM |
| ATP (Sigma) | 1.0 mM | 0.2-5 mM |
| pyruvate oxidase | 10.0 u/ml | 2-30 u/ml |
| peroxidase (Sigma) | 5.0 u/ml | 1-15 u/ml |
| phosphate buffer (pH 7.5) | 20 mM | 5-50 mM |
| thiamine pyrophosphate | 0.1 mM | 0.02-0.5 mM |
| $MgCl_2$ | 5 mM | 1-15 mM |
| 4-aminoantipyrine | 0.03% | 0.01-0.2% |
| phenol | 0.02% | 0.01-0.15% |
| Triton X-100 | 0.1% | 0.05-0.3% |

* Other buffer solution (pH 6.5-8) can be used.

Blank for reagent is measured at 10°C with absorbancy at 500 nm after aseptically filtered the incubation mixture hereinabove using milipore filter.

Following examples illustrate the present invention but are not construed as limiting.

Example 1

(Isolation of chromosomal DNA):

Chromosomal DNA of Aerococcus viridans IFO 012219 was isolated by following procedure.

The strain was cultured overnight with shaking in a bouillon medium (150 ml, containing 0.5% sodium thiosulfate) at 37°C. Cultured cells were collected by centrifugation at 3,000 rpm for 10 min. Lysozyme solution (10 mg/ml, 1 ml) was added to a suspension of the cells in a solution of 10% sucrose, 50 mM Tris-HCl (pH 8.0) and 50 mM EDTA, and incubated at 37°C for 15 min., further added 10% SDS (sodium dodecyl sulfate)(1 ml). Equal volume of a mixture of chloroform-phenol (1:1) was added to the suspension, mixed with stirring, centrifuged at 10,000 rpm for 3 min. to separate aqueous layer and solvent layer. Aqueous layer was collected. Two-fold in volume of ethanol was gently added to the aqueous layer to make double phase, and DNA was collected by winding on a glass rod with gentle stirring. Collected DNA was dissolved in a solution (10 ml) consisting of 10 mM Tris+HCl (pH 8.0) and 1 mM EDTA (hereinafter designated as TE solution). A solution was treated with an equal volume of a mixture of chloroform - phenol and centrifuged to separate an aqueous layer. Two-fold in volume of ethanol was added to the aqueous layer and DNA was isolated by the same way as above, then dissolved in the TE solution (2 ml).

Example 2

(Separation of pACYC 184 plasmid DNA):

A strain of Escherichia coli pM 191 [J. Bacteriol., 134:1141 (1981), ATCC 37033] carrying pACYC 184 was cultured with shaking in BHI medium (Difco, 1 lit.). When growth of bacterial cells measured by turbidity was observed as $OD_{660}$ = 1.0 spectinomycin (final concentration 300 µg/ml, plasmid resistant marker is chloramphenicol) was added, and continued to shake at 37°C for 16 hours. Grown cells were collected at 3,000 rpm for 10 min., and a plasmid DNA was prepared according to the method of lysozyme - SDS and cesium chloride - ethidium bromide [Maniortis et al., Molecular Cloning, pp 86-94, Cold Spring Harbor (1982)].

Example 3

[Preparation of plasmid pOXI3 containing pyruvate oxidase (POP) gene]:

(i) Chromosomal DNA (2 µl, approximately 0.5 µg) of A. viridans prepared in Example 1 was mixed with EcoRI cleavage buffer (1 µl) (500 mM Tris-HCl, pH 7.5, 70 ml $MgCl_2$, 1 M NaCl and 70 mM mercaptoethanol), E CoRI (1 µl:Takara Syuzo Co., Ltd) and water (6 µl), and cleaved at 37°C for 1 hour. Separately prepared plasmid pACYC 184 DNA (ca 0.3 µg) was cleaved in the same way with EcoRI. Alkaline phosphatase (hereinafter designated as BAP, Takara Shuzo Co.) (0.6 unit) was added thereto and incubated at 65°C for 1 hour. The EcoRI-treated two kinds of DNA were mixed, 3M sodium acetate added (1/10 volume), the mixture further treated with an equal volume of a mixture of chloroform and phenol, and the aqueous layer was collected by centrifugation.

Two-fold in volume of ethanol was added to the aqueous layer, which was centrifuged to precipitate the DNA which was dried in vacuo. Lyophilized DNA was dissolved in water (89 µl). Ten times conc. ligation buffer (0.5 M Tris-HCl, pH 7.6, 0.1 M $MgCl_2$, 0.1 M dithiothreitol, 10 mM spermidine and 10 mM ATP)(10 µl) and T4 DNA ligase (1 µl, Takara Shuzo Co., 175 units) were added thereto, mixed and let stand at 4°C for overnight. The said DNA solution was treated with chloroform - phenol, DNA collected by precipitation with ethanol,dried in vacuo and dissolved in TE (10 µl).

(ii) Logarithmically growing Escerichia coli W3110 (obtained from National Institute of Heredity, Japan, stock No. ME 7778, ATCC 27325) in BHI medium (100 ml, Brain Heart Infusion, Difco) was collected by centrifugation at 10,000 rpm for 2 min., and suspended in an ice cold solution (40 ml, pH 5.8) consisting of 30 mM potassium acetate, 100 mM RbCl, 10 mM $CaCl_2$, 50 mM $MnCl_2$ and 15% glycerine. The suspension was let stand for 5 min. at 0°C, centrifuged and discarded the supernatant. Further precipitate was suspended in a solution (4 ml, pH 6.5) consisting of 10 mM MOPS buffer (Dotite Co.), 75 mM $CaCl_2$, 10 mM RbCl and 15% glycerine, and let stand for 15 min, at 0°C to prepare competent cells.

(iii) DNA solution (10 µl) prepared as described in (i) was added to the E. coli suspension from (ii) (200µl) and let stand for 30 min. at 0°C. BHI medium (1 ml) was added thereto, kept at 37°C for 90 min. and 100 µl thereof was spread over BHI agar plate containing tetracycline (15 µg/ml), then cultured at 37°C overnight to obtain a transformant. The transformant cells were replicated on a POP medium plate (peptone 5 g, meat extract 2 g, yeast extract 5 g, NaCl 1 g, $K_2HPO_4$ 1 g, $MgSO_4$ 0.5 g, peroxidase 500 IU, FAD 7.85 mg, dianisidine 0.1 g, thiaminepyrophosphate 42.4 mg, pyruvate 1 mℓ, agar 15 g, distilled water 1 lit., pH 7.0) and further cultured at 37°C overnight. Approximately 4,500 colonies of transformant were checked, and one colony was obtained the surrounding of which was brownish brown. This strain was designated as Escherichia coli W3110-pOXI3 and was deposited as FERM P-No. 9071 (FERM BP-1565).

The strain when cultured at 37°C overnight in BHI medium was shown to yield 3 units/ml of pyruvate oxidase.

A plasmid in this strain was isolated according to the method in Example 2 and was designated as pOXI3 which contains pyruvate oxidase gene and pACYC 184 gene.

Example 4

[Mapping of pOXI3 and determination of base sequence of POP gene]:

a pOXI3 plasmid DNA was prepared from E. coli W3110 pOXI3 according to the same procedure as described in Example 2 for the preparation of pACYC 184.

A restriction map of pOXI3 DNA by digestion with restriction enzymes ClaI, EcoRV, HindIII, ScaI, PstI, PvuII, XbaI (Takara Shuzo Co.) and HpaI (Toyobo Co.) was prepared with the result shown in Fig. 1.

Base sequence of DNA encoding pyruvate oxidase gene was determined by dideoxy method (Science, 214: 1205-1210, 1981) using M13 phage. In Fig. 2-1, 2-2 and 2-3, the base sequence of POP structural gene and the amino acid sequence of the polypeptide for which the gene codes are illustrated.

Example 5

[Production of pyruvate oxidase]:

E. coli W3110 pOXI3 was cultured in BHI medium (Difco)(20 lit.) at 37°C for 18 hours in a jar-fermenter, and centrifuged at 5,000 rpm for 10 min. to collect the cells. Bacterial cells were washed with physiological saline (2 lit.) and suspended in 10 mM phosphate buffer (pH 7.0). Lysozyme (1 mg/ml), EDTA.2Na (2 mM) and Triton X-100 (0.1%) were added and kept and 37°C for 30 min. then centrifuged at 5,000 rpm for 10 min. to separate the supernatant solution.

Ammonium sulfate was added to the supernatant (1.9 ℓ) at 40~66% saturation and the precipitate was collected by centrifugation (5,000 rpm, 10 min.). The precipitate was dissolved in 10 mM phosphate buffer (pH 7.0, containing 10 μMFAD)(200 ml), then desalted by Sephadex G-25. The desalted enzyme solution was charged on a column of DEAE-Sepharose CL-6B for ion exchange chromatography and the active fraction was collected, desalted and lyophilized to obtain an enzyme powder. The enzyme shows 51 u/mg of activity.

Example 6

[Determination of N-terminal amino acid sequence of pyruvate oxidase]:

Amino acid sequence of 10 amino acids from N-terminal of pyruvate oxidase obtained in Example 5 was determined by amino acid sequencer (Beckman System 980 ME).

Approximately 80% thereof contains Met at the N-terminal and the sequence from amino acid position 1 is shown as "Ser, Asp, Asn, Lys, Ile, Asn, Ile, Gly, Leu, (Ala)".

Example 7

Blank value of reagent in pyruvate oxidase with contaminant ATP-ase using ADP coloring reagent prepared in (2) hereinbefore is observed. As shown in Fig. 3, an increase in the blank value caused by generating ADP from ATP by contaminant ATP-ase was observed with known pyruvate oxidase. No increase in the blank value was observed in pyruvate oxidase of the present invention with contaminant ATP-ase of 0.0005%.

Example 8

Reagents of the following composition containing pyruvate oxidase with contaminant ATP-ase of 0.005% and 0.0005% were prepared, and triglyceride in serum was comparatively measured by using the reagent.

| | |
|---|---|
| 0.2 M PIPES-NaOH (pH 7.3) | 0.3 ml |
| 0.1 M ATP | 0.15 ml |
| 0.1 M $MgCl_2$ | 0.15 ml |
| 10 mM thiaminepyrophosphate | 0.3 ml |
| 0.3% TOOS | 0.3 ml |
| 0.2% 4-aminoantipyrine | 0.3 ml |
| peroxidase (45 u/ml) | 0.3 ml |
| glycerokinase (25 u/ml) | 0.05 ml |
| 1 mM FAD | 0.05 ml |
| 10 mM phosphate buffer (pH 7.3) | 0.15 ml |
| pyruvate oxidase (500 u/ml) | 0.03 ml |
| 0.1 M phosphoenolpyruvate | 0.03 ml |
| pyruvate kinase (500 u/ml) | 0.03 ml |
| lipase (3,000 u/ml) | 0.03 ml |
| $H_2O$ | 0.56 ml |

[Experimental method]

Human serum (20-100 µl) was added to the triglyceride coloring reagent hereinabove (3.0 ml), which is either one immediately after preparation or the other stored for 20 hours at room temperature in the dark room. The mixture was incubated at 37°C for 15 min. then immediately measured the absorbance at 550 nm. Result is shown in Fig. 4.

In the figure:

●-●: immediately after preparation of the reagent with pyruvate oxidase of contaminant 0.005% ATP-ase.

●--●: immediately after preparation of the reagent with pyruvatge oxidase of contaminant 0.0005% ATP-ase.

○-○: 20 hours stored reagent with pyruvate oxidase of contaminant 0.005% ATP-ase.

○--○: 20 hours stored reagent with pyruvate oxidase of contaminant 0.0005% ATP-ase.

Example 9

Reagents of the following composition containing pyruvate oxidase with contaminant ATP-ase of 0.005% and 0.0005% were prepared, and hexokinase activity is measured.

| | |
|---|---|
| 0.2 M HEPES-NaOH (pH 7.6) | 0.3 ml |
| 0.1 M ATP | 0.15 ml |
| 2 M glucose | 0.30 ml |
| 0.1 M $MgCl_2$ | 0.24 ml |
| 10 mM thiaminepyrophosphate | 0.30 ml |
| 0.3% TOOS | 0.30 ml |
| 0.2% 4-aminoantipyrine | 0.30 ml |
| peroxidase (45 u/ml) | 0.30 ml |
| 10 mM phosphate buffer (pH 7.6) | 0.15 ml |
| pyruvate oxidase (500 u/ml) | 0.30 ml |
| 0.1 M phosphoenolpyruvate | 0.03 ml |
| pyruvate kinase (500 u/ml) | 0.03 ml |
| $H_2O$ | 0.57 ml |

[Experimental method]

Hexokinase (Oriental Yeast Co.)(50 µl) was added to the reagent for hexokinase asay immediately after preparation or 20 hours stored in dark place at room temperature, and continuously measured the absorbancy at 550 nm at 37°C.

Hexokinase activity was calculated by an increase absorption per minute after 3 min. of enzyme addition. (Fig. 5)

In Fig. 5:

●-●: immediately after preparation of the reagent with pyruvate oxidase of contaminant 0.005% ATP-ase.

●--●: immediately after preparation of the reagent with pyruvate oxidase of contaminant 0.0005% ATP-ase.

O-O: 20 hours stored reagent with pyruvate oxidase of contaminant 0.005% ATP-ase.

O--O: 20 hours stored reagent with pyruvate oxidase of contaminant 0.0005% ATP-ase.

As shown hereinabove a reagent for ADP assay prepared by adding pyruvate oxidase of contaminant less than 0.0005% provides low increased blank value of coloring reagent and hence long term storage of the reagent can be achieved.

Example 10

Human serum (20 µl, three types of sample A, B and C) was added to the reagent for GOT activity assay (1.0 ml) of the following composition, and incubated at 37°C for 30 min. Reaction ws stopped by adding McIlvain buffer (pH 5.5, 2.0 ml) containing 1% Triton X-100 and 0.1 M EDTA and the absorbance at 550 nm was measured. Reaction solutions without adding L-aspartate and α-ketoglutarate were prepared and treated as above for blank assay.

| | |
|---|---|
| 0.5 M HEPES-NaOH (pH 7.0) | 0.08 ml |
| 0.2 M $KH_2PO_4$-NaOH (pH 7.0) | 0.02 ml |
| 0.2 M L-aspartate (pH 7.0) | 0.5 ml |
| 0.2 M α-ketoglutarate (pH 7.0) | 0.05 ml |
| 20 mM thiaminepyrophosphate | 0.05 ml |
| 0.5 M $MgCl_2$ | 0.01 ml |
| 20 mM 4-aminoantipyrine | 0.05 ml |
| 20 mM TOOS | 0.05 ml |
| peroxidase (45 u/ml) | 0.05 ml |
| pyruvate oxidase (160 u/ml) | 0.05ml |
| oxaloacetate decarboxylase | 0.05 ml |
| $H_2O$ | 0.04 ml |

| Human serum | | A | B | C |
|---|---|---|---|---|
| lactate | | 1.3 | 3.1 | 1.9 |
| prior known pyruvate oxidase | absorbance | 0.036 | 0.072 | 0.046 |
| | blank | 0.015 | 0.036 | 0.022 |
| | difference | 0.021 | 0.046 | 0.024 |
| pyruvate oxidase of the present invention | absorbance | 0.022 | 0.045 | 0.025 |
| | blank | 0 | 0 | 0.001 |
| | difference | 0.022 | 0.045 | 0.024 |

As shown in the result, an increase in the absorbance in blank assay was observed for using prior known pyruvate oxidase due to serum lactate interference, and so difference in absorbance should carefully be determined. On the contrary, no increase in blank assay was observed for using pyruvate oxidase of the present invention, and so pyruvate oxidase of the present invention is not contaminated with lactate oxidase and can be used without calculating the difference in absorbance between assay and blank-value.

According to the present invention, base sequence of pyruvate oxidase gene and amino acid sequence of pyruvate oxidase were provided. Also a process for production of pyruvate oxidase applying genetic engineering technique was provided.

Further, assay method using pyruvate oxidase of the present invention shows almost no increase in blank-value and so the long term storage of reagent can be achieved.

**Claims**

1. A pyruvate oxidase having the ability to catalyse a reaction from pyruvate, phosphate and oxygen with the formation of acetylphosphate, carbon dioxide and hydrogen peroxide, an ATP-ase content of below 0.0005% and substantially no lactate oxidase activity and comprising the amino acid sequence shown in Fig 2 of the drawings.

2. A polypeptide having pyruvate oxidase activity as defined in claim 1 which polypeptide consists of the amino acid sequence shown in Fig. 2 of the drawings or such a sequence in which the N-terminal Met residue is substituted by another amino acid residue, hydrogen or acetyl, and the other terminal Lys residue is bonded to an amino acid residue, -OH or $-NH_2$.

3. A polydeoxyribonucleic acid which is exogenous and has a base sequence coding for a polypeptide as claimed in claim 2.

4. A polydeoxyribonucleic acid according to claim 3 wherein the base sequence is as shown in Fig. 2 of the drawings or such a sequence in which the 5'-terminal ATG codon is replaced by another codon excepting TAA, TAG or TGA or by hydrogen, and the 3'-terminal AAA codon is attached to a codon or hydrogen.

5. A recombinant vector comprising a polydoxyribonucleic acid as claimed in claim 3 or 4.

6. A vector according to claim 5 which is a plasmid.

7. A vector according to claim 6 wherein the plasmid is pOXI3 having a restriction map as shown in Fig. 1.

8. A transformant comprising a microorganism transformed with a vector as claimed in claim 5, 6, or 7.

9. A transformant according to claim 8 wherein the microorganism is a strain of Escherichia coli.

10. A transformant according to claim 9 which is Escherichia coli W3110 pOXI3 (FERM BP-1565).

11. A process for producing pyruvate oxidase which comprises culturing a transformant as claimed in claim 8, 9 or 10, and isolating a polypeptide consisting of or comprising pyruvate oxidase from the product of cultivation.

12. An assay method for ADP which comprises:
    (a) generating ATP and pyruvate from ADP and phosphoenol pyruvate by transphosphorylation using pyruvate kinase.
    (b) causing detectable changes by generating acetylphosphate, carbon dioxide and hydrogen peroxide by action of a pyruvate oxidase as claimed in claim 1 on pyruvate generated in step (a), and
    (c) detecting the said detectable changes by acting enzyme and reagent.

13. An assay method according to claim 12 wherein said ADP is an existing ADP in an ADP-generating reaction or ADP in an ADP-consuming reaction.

**14.** An assay method according to claim 13 wherein said ADP-generating reaction is selected from the following:

1 a reaction of hexokinase;
ATP + D-hexose → ADP + D-hexose-6-phosphate
2 a reaction of glucokinase;
ATP + D-glucose → ADP + D-glucose-6-phosphate
3 a reaction of amylase (with maltase);
glucose polymer (soluble starch, amylose or other oligosaccharide or derivatives thereof) + $nH_2O$ → D-glucose + maltose, and
maltose + $2H_2O$ → 2 D-glucose
ATP + D-glucose → ADP + D-glucose-6-phosphate
4 a reaction of adenosine kinase;
ATP + adenosine → ADP + AMP
5 a reaction of thymidine kinase;
ATP + thymidine → ADP + thymidine-5′-phosphate
6 a reaction of NAD kinase;
ATP + $NAD^+$ → ADP + $NADP^+$
7 a reaction of enzyme action from NADH + $H^+$ to generate $NAD^+$
NADH + $H^+$ + substrate A (oxydized form) → $NAD^+$ + $H_2A$ and ATP + $NAD^+$ → ADP + $NADP^+$
8 a reaction of riboflavin kinase;
ATP + riboflavin → ADP + flavin -5′-phosphate
9 a reaction of glycerol kinase;
ATP + glycerol → ADP + glycerol-3-phosphate
10 a reaction of triglyceride assay (with lipase);
triglyceride + $3H_2O$ → glycerol + 3 fatty acid,
and ATP + glycerol → ADP + glycerol-3-phosphate
11 a reaction of lipase assay;
di- or triglyceride + $nH_2O$ → glycerol + n fatty acid, and
ATP + glycerol → ADP + glycerol-3- phosphate
12 a reaction of choline kinase;
ATP + choline → ADP + choline phosphate
13 a reaction of choline esterase assay;
choline ester (fatty acid ester or aryl ester; RCOO) + $H_2O$ → choline + RCOO- and
ATP + choline → ASP + choline phosphate
14 a reaction of phospholipid (lecithin) assay (with phospholipase D);
lecithin + $H_2O$ → phosphatidate + choline and
ATP + choline → ADP + choline phosphate
15 a reaction of protein kinase;
ATP + protein → ADP + phospho-protein, and
16 a reaction of creatine kinase;
ATP + creatine → ADP + creatine phosphate.

**15.** An assay method according to claim 13 wherein said ADP-consuming reaction is selected from the following:

1 a reaction of carbamate kinase;
ADP + carbamoyl phosphate → $NH_3$ + ATP + $CO_2$
2 a reaction of phosphoglycerate kinase;
ADP + D-1,3-bisphosphoglycerate → ATP + 3-glycerol-D-glycetrate
3 a reaction of formate kinase;
ADP + formyl phosphate → ATP + formate
4 a reaction of creatine kinase;
ADP + creatine phosphate → ATP + creatine
5 a reaction of ammonia kinase;
ADP + phosphoramide → ATP + $NH_4^+$
6 a reaction of myokinase:
2ADP → ATP + AMP.

**16.** An assay method for ATP which comprises:

(a) generating ADP and phosphate compound from ATP and non-phosphate compound by transphosphorylation using kinase,

(b) generating ATP and pyruvate from ADP generated in step (a) and phosphenol pyruvate by transphosphorylation using pyruvate kinase,

(c) causing detectable changes by generating acetylphosphate, carbon dioxide and hydrogen peroxide by an action of a pyruvate oxidase as claimed in claim 1 on pyruvate generated in step (b), and

(d) detecting the said detectable changes by acting enzyme and reagent.

**17.** An assay method according to claim 16 wherein said ATP is an existing ATP, ATP in an ATP-consuming reaction or ATP in an ATP-generating reaction.

**18.** An assay method according to claim 17 wherein said ATP-consuming reaction is selected from the following:

1 a reaction of methionine-adenosyl transferase;
ATP + L-methionine + $H_2O$ → S-adenosyl-L-methionine + PPi + Pi
2 a reaction of hexokinase;
ATP + D-hexose → ADP + D-hexose-6-phosphate
3 a reaction of glucokinase;
ATP + D-glucose → ADP + D-glucose-6-phosphate
4 a reaction of amylase (with maltase);
glucose polymer (soluble starch, amylose or other oligosaccaride or derivatives thereof) + $nH_2O$ → D-glucose + maltose, and
maltose + $2H_2O$ → 2 D-glucose
ATP + D-glucose → ADP + D-glucose-6-phosphate
5 a reaction of adenosine kinase;
ATP + adenosine → ADP + AMP
6 a reaction of thymidine kinase;
ATP + thymidine → ADP + thymidine-5′-phosphate
7 a reaction of NAD kinase;
ATP + $NAD^+$ → ADP + $NADP^+$
8 a reaction of enzyme action from NADH + $H^+$ to generate $NAD^+$
NADH + $H^+$ + substrate A (oxydized form) → $NAD^+$ + $H_2A$ and ATP + $NAD^+$ → ADP + $NADP^+$
9 a reaction of riboflavin kinase;
ATP + riboflavin → ADP + flavin -5′-phosphate
10 a reaction of glycerol kinase;
ATP + glycerol → ADP + glycerol-3-phosphate
11 a reaction of triglyceride assay (with lipase);
triglyceride + $3H_2O$ → glycerol + 3 fatty acid,
and ATP + glycerol → ADP + glycerol-3-phosphate
12 a reaction of lipase assay;
di- or triglyceride + $nH_2O$ → glycerol + n fatty acid, and
ATP + glycerol → ADP + glycerol-3-phosphate
13 a reaction of choline kinase;
ATP + choline → ADP + choline phosphate
14 a reaction of choline esterase assay;
choline ester (fatty acid ester or aryl ester; RCOO) + $H_2O$ → choline + $RCOO^-$ and
ATP + choline → ADP + choline phosphate
15 a reaction of phospholipid (lecithin) assay (with phospholipase D);
lecithin + $H_2O$ → phosphatidate + choline and ATP + choline → ADP + choline phosphate
16 a reaction of protein kinase:
ATP + protein → ADP + phospho-protein
17 a reaction of creatine kinase;
ATP + creatine → ADP + creatine phosphate

**19.** An assay method for a non-phosphate compound which comprises:

a) generating ADP and phosphate compound from ATP and non-phosphate compound by transphosphorylation using kinase,

(b) generating ATP and pyruvate from ADP generated in step (a) and phosphoenol pyruvate by transphosphorylation using pyruvate kinase,
(c) causing detectable changes by generating acetylphosphate, carbon dioxide and hydrogen peroxide by action of a pyruvate oxidase as claimed in claim 1 on pyruvate generated in step (b), and
(d) detecting the said detectable changes by acting enzyme and reagent.

**Patentansprüche**

**1.** Pyruvat-Oxidase mit der Fähigkeit, die Umsetzung von Pyruvat, Phosphat und Sauerstoff unter Bildung von Acetylphosphat, Kohlendioxid und Wasserstoffperoxid zu katalysieren, einem ATPase-Gehalt von weniger als 0,0005% und im wesentlichen fehlender Lactatoxidase-Aktivität, und umfassend die Aminosäuresequenz gemäß Figur 2 der Figuren.

**2.** Polypeptid mit Pyruvat-Oxidase-Aktivität nach Anspruch 1, dadurch **gekennzeichnet,** daß das Polypeptid aus der in Figur 2 der Figuren gezeigten Aminosäuresequenz oder einer solchen Sequenz, worin der N-terminale Met-Rest durch einen anderen Aminosäurerest, ein Wasserstoffatom oder eine Acetylgruppe substituiert ist, und der andere terminale Lys-Rest an einen Aminosäurerest, -OH oder $-NH_2$ gebunden ist, besteht.

**3.** Polydesoxyribonucleinsäure, die exogen ist und eine Basensequenz, die ein Polypeptid nach Anspruch 2 codiert, besitzt.

**4.** Polydesoxyribonucleinsäure nach Anspruch 3, dadurch **gekennzeichnet,** daß die Basensequenz, wie in Figur 2 der Figuren gezeigt, ist oder eine solche Sequenz ist, in der das 5'-terminale ATG-Codon durch ein anderes Codon, ausgenommen TAA, TAG oder TGA, oder durch Wasserstoff ersetzt ist, und das 3'-terminale AAA-Codon an ein Codon oder ein Wasserstoffatom gebunden ist.

**5.** Rekombinanter Vektor, umfassend eine Polydesoxyribonucleinsäure nach Anspruch 3 oder 4.

**6.** Vektor nach Anspruch 5, dadurch **gekennzeichnet,** daß er ein Plasmid ist.

**7.** Vektor nach Anspruch 6, dadurch **gekennzeichnet,** daß das Plasmid pOXI3 mit der Restriktionskarte nach Figur 1 ist.

**8.** Transformant, umfassend einen Mikroorganismus, der mit einem Vektor nach Anspruch 5, 6 oder 7 transformiert worden ist.

**9.** Transformant nach Anspruch 8, dadurch **gekennzeichnet,** daß der Mikroorganismus ein Escherichia coli-Stamm ist.

**10.** Transformant nach Anspruch 9, dadurch **gekennzeichnet,** daß er Escherichia coli W3110 pOXI3 (FERM BP-1565) ist.

**11.** Verfahren zur Produktion von Pyruvat-Oxidase, dadurch **gekennzeichnet,** daß man einen Transformanten nach Anspruch 8, 9 oder 10 züchtet und ein Polypeptid, das aus einer Pyruvat-Oxidase besteht oder sie enthält, aus dem Züchtungsprodukt isoliert.

**12.** Assay-Verfahren auf ADP, dadurch **gekennzeichnet,** daß man
(a) ATP und Pyruvat aus ADP und Phosphoenol-Pyruvat durch Transphosphorylierung unter Verwendung von Pyruvat-Kinase erzeugt,
(b) nachweisbare Veränderungen durch Erzeugung von Acetylphosphat, Kohlendioxid und Wasserstoffperoxid durch Wirkung einer Pyruvat-Oxidase nach Anspruch 1 an dem in Stufe (a) erzeugten Pyruvat hervorruft, und
(c) die nachweisbaren Veränderungen durch Wirkung eines Enzyms und eines Reagenses nachweist.

**13.** Assay-Verfahren nach Anspruch 12, dadurch **gekennzeichnet,** daß das ADP ein in einer ADP-erzeugenden Reaktion vorhandenes ADP oder ein ADP in einer ADP-verbrauchenden Reaktion ist.

**14.** Assay-Verfahren nach Anspruch 13, dadurch **gekennzeichnet,** daß die ADP-erzeugende Reaktion aus den folgenden Reaktionen ausgewählt ist:
1. Hexokinasereaktion;
ATP + D-Hexose → ADP + D-Hexose-6-phosphat
2. Glucokinasereaktion;
ATP + D-Glucose → ADP + D-Glucose-6-phosphat
3. Amylasereaktion (mit Maltase);
Glucosepolymeres (lösliche Stärke, Amylose oder ein anderes Oligosaccharid oder Derivate davon) + $nH_2O$ → D-Glucose + Maltose, und
Maltose + $2H_2O$ → 2 D-Glucose
ATP + D-Glucose → ADP + D-Glucose-6-phosphat
4. Adenosinkinasereaktion;
ATP + Adenosin → ADP + AMP
5. Thymidinkinasereaktion;
ATP + Thymidin → ADP + Thymidin-5'-phosphat
6. NAD-Kinasereaktion;
ATP + $NAD^+$ → ADP + $NADP^+$
7. Enzymatische Reaktion aus NADH + $H^+$ zur Erzeugung von $NAD^+$;
NADH + $H^+$ + Substrat A (oxidierte Form) → $NAD^+$ + $H_2A$ und ATP + $NAD^+$ → ADP + $NADP^+$
8. Riboflavinkinasereaktion;
ATP + Riboflavin → ADP + Flavin-5'-phosphat
9. Glycerinkinasereaktion;
ATP + Glycerin → ADP + Glycerin-3-phosphat
10. Triglyceridassayreaktion (mit Lipase);
Triglycerid + $3H_2O$ → Glycerin + 3 Fettsäuren und ATP + Glycerin → ADP + Glycerin-3-phosphat
11. Lipaseassayreaktion;
Di- oder Triglycerid + $nH_2O$ → Glycerin + n Fettsäure, und
ATP + Glycerin → ADP + Glycerin-3-phosphat
12. Cholinkinasereaktion;
ATP + Cholin → ADP + Cholinphosphat
13. Cholinesteraseassayreaktion;
Cholinester (Fettsäureester oder Arylester; RCOO) + $H_2O$ → Cholin + RCOO- und
ATP + Cholin → ADP + Cholinphosphat
14. Phospholipid(Lecithin)assayreaktion (mit Phospholipase D);
Lecithin + $H_2O$ → Phosphatidat + Cholin und
ATP + Cholin → ADP + Cholinphosphat
15. Proteinkinasereaktion;
ATP + Protein → ADP + Phosphoprotein, und
16. Creatinkinasereaktion;
ATP + Creatin → ADP + Creatinphosphat.

**15.** Assay-Verfahren nach Anspruch 13, dadurch **gekennzeichnet,** daß die ADP-verbrauchende Reaktion aus den folgenden Reaktionen ausgewählt ist:
1. Carbamatkinasereaktion;
ADP + Carbamoylphosphat → $NH_3$ + ATP + $CO_2$
2. Phosphoglyceratkinasereaktion;
ADP + D-1,3-Bisphosphoglycerat → ATP + 3-Glycerin-D-glycerat
3. Formeatkinasereaktion;
ADP + Formylphosphat → ATP + Formeat
4. Creatinkinasereaktion;
ADP + Creatinphosphat → ATP + Creatin
5. Ammoniumkinasereaktion;
ADP + Phosphoramid → ATP + $NH_4^+$
6. Myokinasereaktion;
2ADP → ATP + AMP.

**16.** Assay-Verfahren auf ATP, dadurch **gekennzeichnet,** daß man
(a) ADP und eine Phosphatverbindung aus ATP und einer Nicht-Phosphatverbindung durch Transphosphorylierung unter Verwendung einer Kinase erzeugt,
(b) ATP und Pyruvat aus dem in Stufe (a) erzeugten ADP und Phosphoenolpyruvat durch Transphosphorylierung unter Verwendung einer Pyruvat-Kinase erzeugt,
(c) nachweisbare Veränderungen durch Erzeugung von Acetylphosphat, Kohlendioxid und Wasserstoffperoxid durch Wirkung einer Pyruvat-Oxidase nach Anspruch 1 an dem in Stufe (b) erzeugten Pyruvat hervorruft, und
(d) die nachweisbaren Veränderungen durch Wirkung eines Enzyms und eines Reagenses nachweist.

**17.** Assay-Verfahren nach Anspruch 16, dadurch **gekennzeichnet,** daß das ATP ein vorhandenes ATP, ATP in einer ATP-verbrauchenden Reaktion oder ATP in einer ATP-erzeugenden Reaktion ist.

**18.** Assay-Verfahren nach Anspruch 17, dadurch **gekennzeichnet,** daß die ATP-verbrauchende Reaktion aus den folgenden Reaktionen ausgewählt ist:
1. Methionin-Adenosyltransferasereaktion;
ATP + L-Methionin + $H_2O$ → S-Adenosyl-L-methionin + $PP_i$ + $P_i$
2. Hexokinasereaktion;
ATP + D-Hexose → ADP + D-Hexose-6-phosphat
3. Glucokinasereaktion;
ATP + D-Glucose → ADP + D-Glucose-6-phosphat
4. Amylasereaktion (mit Maltase);
Glucosepolymeres (lösliche Stärke, Amylose oder ein anderes Oligosaccharid oder Derivate davon) + $nH_2O$ → D-Glucose + Maltose, und
Maltose + $2H_2O$ → 2 D-Glucose
ATP + D-Glucose → ADP + D-Glucose-6-phosphat
5. Adenosinkinasereaktion;
ATP + Adenosin → ADP + AMP
6. Thymidinkinasereaktion;
ATP + Thymidin → ADP + Thymidin-5'-phosphat
7. NAD-Kinasereaktion;
ATP + $NAD^+$ → ADP + $NADP^+$
8. Enzymatische Reaktion aus NADH + $H^+$ unter Erzeugung von $NAD^+$;
NADH + $H^+$ + Substrat A (oxidierte Form) → $NAD^+$ + $H_2A$ und ATP + $NAD^+$ → ADP + $NADP^+$
9. Riboflavinkinasereaktion;
ATP + Riboflavin → ADP + Flavin-5'-phosphat
10. Glycerinkinasereaktion;
ATP + Glycerin → ADP + Glycerin-3-phosphat
11. Triglyceridassayreaktion (mit Lipase);
Triglycerid + $3H_2O$ → Glycerin + 3 Fettsäure, und
ATP + Glycerin → ADP + Glycerin-3-phosphat
12. Lipaseassayreaktion;
Di- oder Triglycerid + $nH_2O$ → Glycerin + n Fettsäure, und
ATP + Glycerin → ADP + Glycerin-3-phosphat
13. Cholinkinasereaktion;
ATP + Cholin → ADP + Cholinphosphat
14. Cholinesteraseassayreaktion;
Cholinester (Fettsäureester oder Arylester; RCOO) + $H_2O$ → Cholin + $RCOO^-$ und
ATP + Cholin → ADP + Cholinphosphat
15. Phospholipid(Lecithin)assayreaktion (mit Phospholipase D);
Lecithin + $H_2O$ → Phosphatidat + Cholin und
ATP + Cholin → ADP + Cholinphosphat
16. Proteinkinasereaktion;
ATP + Protein → ADP + Phospho-Protein
17. Creatinkinasereaktion;
ATP + Creatin → ADP + Creatinphosphat.

**19.** Assay-Verfahren auf eine Nicht-Phosphatverbindung, dadurch **gekennzeichnet,** daß man
(a) ADP und eine Phosphatverbindung aus ATP und einer Nicht-Phosphatverbindung durch Transphosphorylierung unter Verwendung von Kinase erzeugt,
(b) ATP und Pyruvat aus dem in Stufe (a) erzeugten ADP und Phosphoenolpyruvat durch Transphosphorylierung unter Verwendung von Pyruvat-Kinase erzeugt,
(c) nachweisbare Veränderungen durch Erzeugung von Acetylphosphat, Kohlendioxid und Wasserstoffperoxid durch Wirkung einer Pyruvat-Oxidase nach Anspruch 1 auf das in Stufe (b) erzeugte Pyruvat hervorruft, und
(d) die nachweisbaren Veränderungen durch Wirkung eines Enzyms und eines Reagenses nachweist.

**Revendications**

**1.** Pyruvate oxydase ayant la capacité de catalyser une réaction à partit de pyruvate, phosphate et oxygène avec la formation d'acétylphosphate, de dioxyde de carbone et de péroxyde d'hydrogène, un contenu en ATP-ase inférieur à 0,0005% et substantiellement pas de lactate oxydase et comprenant la séquence acide aminé montrée en figure 2 des dessins.

**2.** Polypeptide ayant une activité pyruvate oxydase, comme définie en revendication 1, lequel polypeptide consiste de la séquence acide aminé montrée en figure 2 des dessins ou d'une séquence dans laquelle le résidu Met N-terminal est substitué par un autre résidu acide aminé, hydrogène ou acétyle , et l'autre résidu Lys terminal est lié à un résidu acide aminé, OH ou $-NH_2$.

**3.** Acide polydésoxyribunocléique qui est exogène et a une séquence de base codante pour un polypeptide comme revendiqué en revendication 2.

**4.** Acide polydésoxyribunocléique selon la revendication 3, dans lequel la séquence de base est comme montrée en figure 2 des dessins ou une séquence dans laquelle le codon ATG 5'-terminal est remplacé par un autre codon, sauf TAA, TAG ou TGA ou pur un hydrogène, et le codon AAA 3'-terminal est lié à un codon ou un hydrogène.

**5.** Vecteur recombinant comprenant un acide polydésoxyribonucléique tel que revendiqué dans les revendications 3 ou 4.

**6.** Vecteur selon la revendication 5, qui est un plasmide.

**7.** Vecteur selon la revendication 6, dans lequel le plasmide est pOX13 ayant une région de restriction comme montrée en figure 1.

**8.** Transformateur comprenant un micro-organisme transformé avec un vecteur, comme revendiqué dans les revendications 5, 6 ou 7.

**9.** Transformateur selon la revendication 8, dans lequel le micro-organisme est une souche de Escherichia coli.

**10.** Transformateur selon la revendication 9, qui est le Escherichia coli W3110 pOX13 (FERM BP-1565).

**11.** Procédé pour produire une pyruvate oxydase, qui comprend la culture d'un transformateur tel que revendiqué en revendication 8, 9 ou 10, et l'isolation d'un polypeptide consistant de ou comprenant une pyruvate oxydase à partir du produit de culture.

**12.** Méthode de test pour ADP qui comprend :
(a) générer de l'ATP et du pyruvate à partir d'ADP et de phosphoénol pyruvate par transphosphorylation, en utilisant une pyruvate kinase.
(b) provoquer des changements détectables par génération d'acétylphosphate, dioxyde de carbone et péroxyde d'hydrogène par action d'une pyruvate oxydase, comme revendiquée dans la revendication 1, sur le pyruvate généré à l'étape (a), et
(c) déecter lesdits changements détectables par action d'un enzyme et d'un réactif.

**13.** Méthode de test selon la revendication 12, dans laquelle ledit ADP est un ADP existant dans une réaction générant de l'ADP ou dans une réaction consommant de l'ADP.

**14.** Méthode de test selon la revendication 13, dans laquelle ladite réaction générant de l'ADP est sélectionnée parmi les suivantes :

1. une réaction d'hexokinase;
ATP + D-hexose → ADP + D-hexose-6-phosphate
2. une réaction de glucokinase;
ATP + D-glucose → ADP + D-glucose-6-phosphate
3. une réaction d'amylase (avec du maltase);
un polymère de glucose (amidon soluble, amylose ou autre oligosaccharide ou dérivés de ceux-ci) + $nH_2O$ → D-glucose + maltose, et
maltose + $2H_2O$ → 2 D-glucose
ATP + D-glucose → ADP + D-glucose-6-phosphate
4. une réaction d'adénosine kinase;
ATP + adénosine → ADP + AMP
5. une réaction de thymidine kinase;
ATP + thymidine → ADP + thymidine-5'-phosphate
6. une réaction de NAD kinase;
ATP + $NAD^+$ → ADP + $NADP^+$
7. une réaction d'action d'enzyme de NADH + $H^+$ en $NAD^+$ généré
NADH + $H^+$ + substrat A (forme oxydée) - $NAD^+$ + $H_2A$ et ATP + $NAD^+$ → ADP + $NADP^+$
8. une réaction de riboflavine kinase;
ATP + riboflavine → ADP + flavin -5'-phosphate
9. une réaction de glycérol kinase;
ATP + glycérol → ADP + glycérol-3-phosphate
10. une réaction de test de triglycéride (avec lipase);
triglycéride + $3H_2O$ → glycérol + 3 acides gras,
et ATP + glycérol → ADP + glycérol-3-phosphate
11. une réaction de test de lipase;
di- ou triglycéride + $nH_2O$ → glycérol + n acide gras, et
ATP + glycérol → ADP + glycérol-3-phosphate
12. une réaction de choline kinase;
ATP + choline → ADP + choline phosphate
13. une réaction de test de choline estérase;
choline ester (ester d'acide gras ou aryl ester; RCOO) + $H_2O$ → choline + RCOO- et
ATP + choline → ADP + choline phosphate
14. une réaction de test de phospholipide (lécithine) (avec une phospholipase D);
lécithine + $H_2O$ → phosphatidate + choline et
ATP + choline → ADP + choline phosphate
15. une réaction de protéine kinase;
ATP + protéine → ADP + phospho-protéine, et
16. une réaction de créatine kinase;
ATP + créatine → ADP + créatine phosphate.

**15.** Méthode de test selon la revendication 13, dans laquelle ladite réaction consommant de l'ADP est choisie parmi les suivantes :

1. une réaction de carbamate kinase;
ADP + carbamoyl phosphate → $NH_3$ + ATP + $CO_2$
2. une réaction de phosphoglycérate kinase;
ADP + D-1,3-bisphosphoglycérate → ATP + 3-glycérol-D-glycétrate
3. une réaction de formate kinase;
ADP + formyl phosphate → ATP + formate
4. une réaction de créatine kinase;
ADP + créatine phosphate → ATP + créatine
5. une réaction d'ammoniac kinase;
ADP + phosphoramide → ATP + $NH_4^+$

6. une réaction de myokinase :
2ADP + ATP + AMP.

**16.** Méthode de test pour ATP qui comprend :
(a) générer de l'ADP et un composé phosphate à partir d'ATP et d'un composé non-phosphate par transphosphorylation, en utilisant une kinase,
(b) générer de l'ATP et du pyruvate à partir d'ADP généré à l'étape (a) et de phosphénol pyruvate par transphosphorylation, an utilisant une pyruvate kinase,
(c) provoquer des changements détectables en générant de l'acétylphosphate, du dioxyde de carbone et du péroxyde d'hydrogène par une action d'une pyruvate oxydase, comme revendiquée dans la revendication 1, sur le pyruvate généré à l'étape (b), et
(d) détecter lesdits changements détectables par action d'un enzyme et d'un réactif.

**17.** Méthode de test selon la revendication 16, dans laquelle ledit ATP est un ATP existant, un ATP dans une réaction consommant de l'ATP ou un ATP dans une réaction générant de l'ATP.

**18.** Méthode de test selon la revendication 17, dans laquelle ladite réaction consommant de l'ATP est choisie parmi les suivantes :
1. une réaction de méthionine-adénosyl transférase;
ATP + L-méthionine + $H_2O$ → S-adénosyl-L-méthionine + PPi + Pi
2. une réaction d'hexokinase;
ATP + D-hexose → ADP + D-hexose-6-phosphate
3. une réaction de glucokinase;
ATP + D-glucose → ADP + D-glucose-6-phosphate
4. une réaction d'amylase (avec un maltase);
polymère de glucose (amidon soluble, amylose ou autre oligosaccharide ou dérivés de ceux-ci) + $nH_2O$ → D-glucose + maltose, et
maltose + $2H_2O$ → 2 D-glucose
ATP + D-glucose → ADP + D-glucose-6-phosphate
5. une réaction d'adénosine kinase;
ATP + adénosine → ADP + AMP
6. une réaction de thymidine kinase;
ATP + thymidine → ADP + thymidine-5'-phosphate
7. une réaction de NAD kinase;
ATP + $NAD^+$ → ADP + $NADP^+$
8. une réaction d'action d'un enzyme à partir de NADH + $H^+$ en $NAD^+$ généré
NADH + $H^+$ + substrat A (forme oxydée) → $NAD^+$ + $H_2A$ et ATP + $NAD^+$ → ADP + $NADP^+$
9. une réaction de riboflavine kinase;
ATP + riboflavine → ADP + flavin-5'-phosphate
10. une réaction de glycérol kinase;
ATP + glycérol → ADP + glycérol-3-phosphate
11. une réaction de test de triglycéride (avec lipase);
triglycéride + $3H_2O$ - glycérol + 3 acide gras,
et ATP + glycérol + ADP + glycérol-3-phosphate
12. une réaction de test de lipase;
di- ou triglycéride + $nH_2O$ → glycérol + n acide gras, et
ATP + glycérol → ADP + glycérol-3-phosphate
13. une réaction de choline kinase,
ATP + choline → ADP + choline phosphate
14. une réaction de test de choline estérase;
choline ester (ester d'acide gras ou aryl ester; RCOO) + $H_2O$ → choline + $RCOO^-$ et
ATP + choline → ADP + choline phosphate
15. une réaction de test de phospholipide (lécithine) (avec phospholipase D);
lécithine + $H_2O$ → phosphatidate + choline et ATP + choline → ADP + choline phosphate
16. une réaction de protéine kinase;
ATP + protéine → ADP + phospho-protéine
17. une réaction de créatine kinase;
ATP + créatine → ADP + créatine phosphate

**19.** Méthode de test pour un composé non-phosphate qui comprend :

(a) générer de l'ADP et un composé phosphate à partir d'ATP et d'un composé non-phosphate par transphosphorylation en utilisant une kinase,

(b) générer de l'ATP et du pyruvate à partir d'ADP généré à l'étape (a) et de phosphoénol pyruvate par transphosphorylation en utilisant une pyruvate kinase,

(c) provoquer des changements détectables par génération d'acétylphosphate, de dioxyde de carbone et de péroxyde d'hydrogène par action d'une pyruvate oxydase, comme revendiquée à la revendication 1, sur le pyruvate généré à l'étape (b), et

(d) détecter lesdits changements détectables par action d'un enzyme et d'un réactif.

FIG. 1

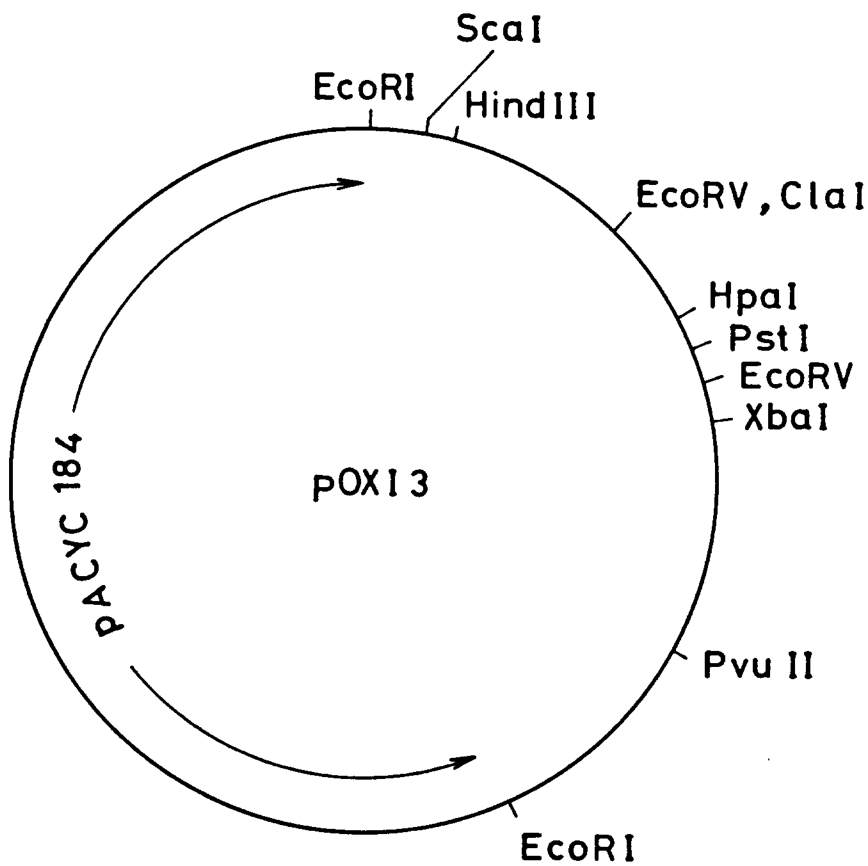
ScaI
EcoRI
HindIII
EcoRV, ClaI
HpaI
PstI
EcoRV
XbaI
pOXI3
pACYC 184
PvuII
EcoRI

# FIG. 2-1

10 20 30 40 50 60
ATGTCAGATAACAAAATTAACATCGGTTTAGCAGTTATGAAGATTTTAGAATCTTGGGGA
MetSerAspAsnLysIleAsnIleGlyLeuAlaValMetLysIleLeuGluSerTrpGly

70 80 90 100 110 120
GCAGATACTATTTATGGTATTCCTTCAGGTACTTTAAGCTCATTAATGGATGCTATGGGT
AlaAspThrIleTyrGlyIleProSerGlyThrLeuSerSerLeuMetAspAlaMetGly

130 140 150 160 170 180
GAAGAAGAAAACAACGTCAAATTCCTACAAGTGAAACACGAAGAAGTAGGTGCAATGGCT
GluGluGluAsnAsnValLysPheLeuGlnValLysHisGluGluValGlyAlaMetAla

190 200 210 220 230 240
GCTGTAATGCAAAGCAAATTCGGCGGTAACTTAGGTGTTACTGTAGGTTCTGGTGGACCA
AlaValMetGlnSerLysPheGlyGlyAsnLeuGlyValThrValGlySerGlyGlyPro

250 260 270 280 290 300
GGTGCATCTCACTTGATCAACGGTTTATACGATGCAGCAATGGATAACATTCCAGTAGTT
GlyAlaSerHisLeuIleAsnGlyLeuTyrAspAlaAlaMetAspAsnIleProValVal

310 320 330 340 350 360
GCGATCTTAGGTTCTCGTCCACAACGCGAATTAAACATGGACGCTTTCCAAGAATTAAAC
AlaIleLeuGlySerArgProGlnArgGluLeuAsnMetAspAlaPheGlnGluLeuAsn

370 380 390 400 410 420
CAGAACCCAATGTACGACCATATTGCAGTTTACAACCGTCGTGTAGCTTATGCTGAGCAA
GlnAsnProMetTyrAspHisIleAlaValTyrAsnArgArgValAlaTyrAlaGluGln

430 440 450 460 470 480
TTACCAAAATTAGTTGACGAAGCAGCTCGTATGGCTATCGCTAAACGCGGTGTAGCAGTT
LeuProLysLeuValAspGluAlaAlaArgMetAlaIleAlaLysArgGlyValAlaVal

490 500 510 520 530 540
CTAGAAGTACCTGGTGATTTTGCTAAAGTTGAAATCGACAACGACCAATGGTATTCATCT
LeuGluValProGlyAspPheAlaLysValGluIleAspAsnAspGlnTrpTyrSerSer

550 560 570 580 590 600
GCAAACAGCTTACGTAAATATGCACCAATCGCTCCAGCAGCACAAGATATCGACGCAGCA
AlaAsnSerLeuArgLysTyrAlaProIleAlaProAlaAlaGlnAspIleAspAlaAla

610 620 630 640 650 660
GTTGAATTATTAAACAACTCTAAACGTCCAGTTATCTACGCTGGTATTGGTACTATGGGC
ValGluLeuLeuAsnAsnSerLysArgProValIleTyrAlaGlyIleGlyThrMetGly

670 680 690 700 710 720
CACGGTCCTGCAGTTCAAGAATTAGCTCGTAAAATCAAAGCGCCAGTTATCACTACTGGT
HisGlyProAlaValGlnGluLeuAlaArgLysIleLysAlaProValIleThrThrGly

# FIG. 2-2

730 740 750 760 770 780
AAAAACTTTGAAACTTTCGAGTGGGATTTCGAAGCGTTAACAGGTTCTACTTATCGTGTA
LysAsnPheGluThrPheGluTrpAspPheGluAlaLeuThrGlySerThrTyrArgVal

790 800 810 820 830 840
GGTTGGAAACCAGCTAACGAAACAATTTTAGAAGCTGACACAGTATTATTTGCTGGTTCA
GlyTrpLysProAlaAsnGluThrIleLeuGluAlaAspThrValLeuPheAlaGlySer

850 860 870 880 890 900
AACTTCCCATTCTCAGAGGTTGAAGGTACTTTCCGTAACGTGGATAACTTCATCCAAATC
AsnPheProPheSerGluValGluGlyThrPheArgAsnValAspAsnPheIleGlnIle

910 920 930 940 950 960
GATATCGACCCAGCTATGTTAGGTAAACGTCACCACGCTGATGTTGCTATCTTAGGTGAT
AspIleAspProAlaMetLeuGlyLysArgHisHisAlaAspValAlaIleLeuGlyAsp

970 980 990 1000 1010 1020
GCTGGTCTAGCAATCGACGAAATCTTAAACAAAGTAGATGCTGTTGAAGAGTCAGCATGG
AlaGlyLeuAlaIleAspGluIleLeuAsnLysValAspAlaValGluGluSerAlaTrp

1030 1040 1050 1060 1070 1080
TGGACAGCTAACTTGAAAAACATCGCTAACTGGCGTGAATACATCAACATGTTAGAAACT
TrpThrAlaAsnLeuLysAsnIleAlaAsnTrpArgGluTyrIleAsnMetLeuGluThr

1090 1100 1110 1120 1130 1140
AAAGAAGAAGGCGACTTGCAATTCTACCAAGTGTACAATGCAATCAACAACCACGCCGAC
LysGluGluGlyAspLeuGlnPheTyrGlnValTyrAsnAlaIleAsnAsnHisAlaAsp

1150 1160 1170 1180 1190 1200
GAAGATGCAATCTACTCTATTGATGTTGGTAACTCAACTCAAACTTCTATCCGTCATTTA
GluAspAlaIleTyrSerIleAspValGlyAsnSerThrGlnThrSerIleArgHisLeu

1210 1220 1230 1240 1250 1260
CATATGACACCTAAAAACATGTGGAGAACTTCTCCATTATTCGCGACAATGGGTATCGCT
HisMetThrProLysAsnMetTrpArgThrSerProLeuPheAlaThrMetGlyIleAla

1270 1280 1290 1300 1310 1320
ATCCCTGGTGGTTTAGGTGCTAAAAACACTTACCCAGATCGTCAAGTTTGGAACATCATC
IleProGlyGlyLeuGlyAlaLysAsnThrTyrProAspArgGlnValTrpAsnIleIle

1330 1340 1350 1360 1370 1380
GGTGATGGTGCTTTCTCTATGACTTACCCAGATGTAGTAACTAACGTACGTTACAACATG
GlyAspGlyAlaPheSerMetThrTyrProAspValValThrAsnValArgTyrAsnMet

1390 1400 1410 1420 1430 1440
CCTGTAATCAACGTTGTATTCTCTAACACTGAATATGCCTTCATCAAAAACAAATATGAA
ProValIleAsnValValPheSerAsnThrGluTyrAlaPheIleLysAsnLysTyrGlu

# FIG. 2-3

```
    1450      1460      1470      1480      1490      1500
GACACTAACAAAAACTTATTCGGTGTAGACTTTACAGATGTTGATTACGCTAAAATCGCT
AspThrAsnLysAsnLeuPheGlyValAspPheThrAspValAspTyrAlaLysIleAla

    1510      1520      1530      1540      1550      1560
GAAGCACAAGGTGCTAAAGGATTTACTGTAAGCCGTATCGAAGATATGGACCGTGTAATG
GluAlaGlnGlyAlaLysGlyPheThrValSerArgIleGluAspMetAspArgValMet

    1570      1580      1590      1600      1610      1620
GCTGAAGCTGTTGCAGCTAATAAAGCAGGTCACACTGTCGTTATCGACTGTAAGATTACT
AlaGluAlaValAlaAlaAsnLysAlaGlyHisThrValValIleAspCysLysIleThr

    1630      1640      1650      1660      1670      1680
CAAGATCGTCCAATCCCTGTAGAAACATTGAAATTAGATTCAAAACTTTACAGCGAAGAC
GlnAspArgProIleProValGluThrLeuLysLeuAspSerLysLeuTyrSerGluAsp

    1690      1700      1710      1720      1730      1740
GAAATCAAAGCTTACAAAGAACGCTACGAAGCTGCTAACTTAGTACCATTCAGAGAGTAC
GluIleLysAlaTyrLysGluArgTyrGluAlaAlaAsnLeuValProPheArgGluTyr

    1750      1760      1770
TTAGAAGCTGAAGGCTTAGAATCTAAATACATCAAA
LeuGluAlaGluGlyLeuGluSerLysTyrIleLys
```

FIG. 3

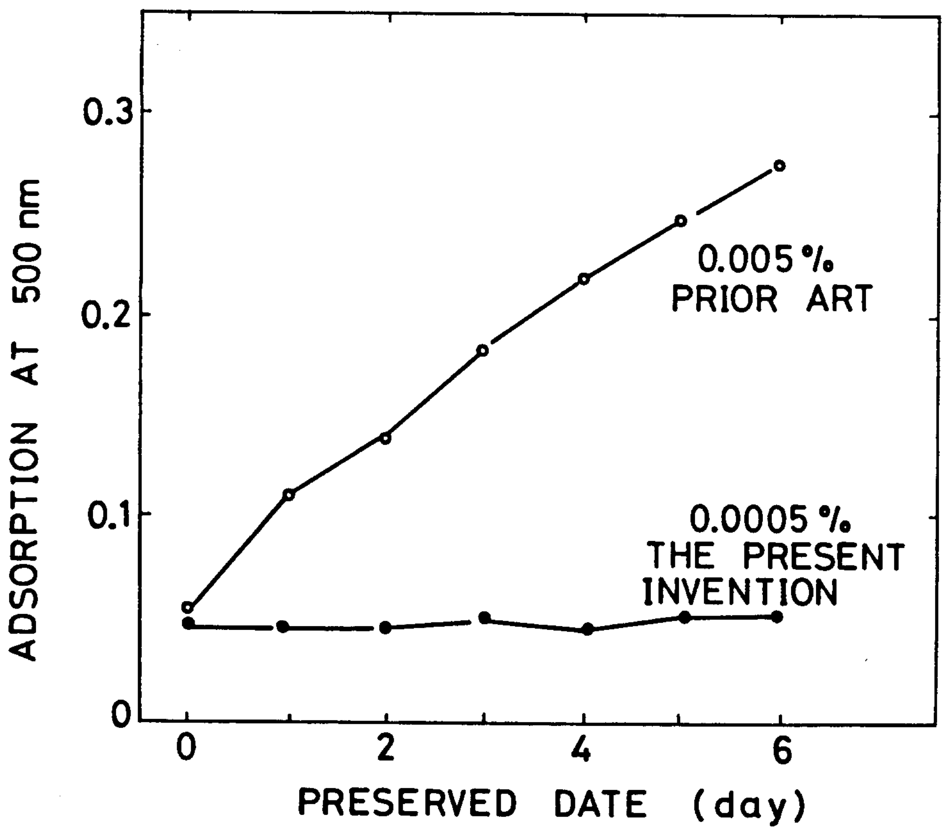
0.3
0.2
0.1
0
ADSORPTION AT 500 nm
0.005 %
PRIOR ART
0.0005 %
THE PRESENT
INVENTION
0
2
4
6
PRESERVED DATE (day)

FIG. 4

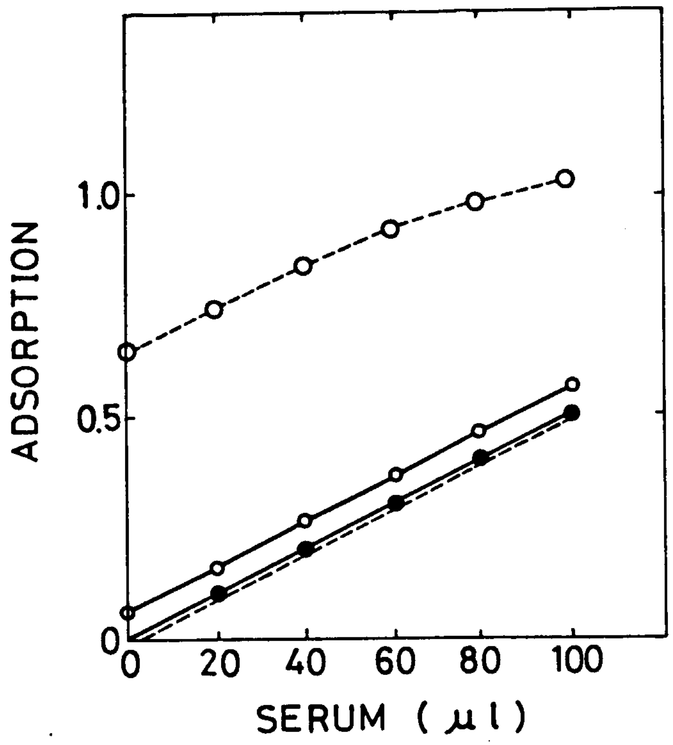
ADSORPTION
1.0
0.5
0
0
20
40
60
80
100
SERUM (μl)

FIG. 5

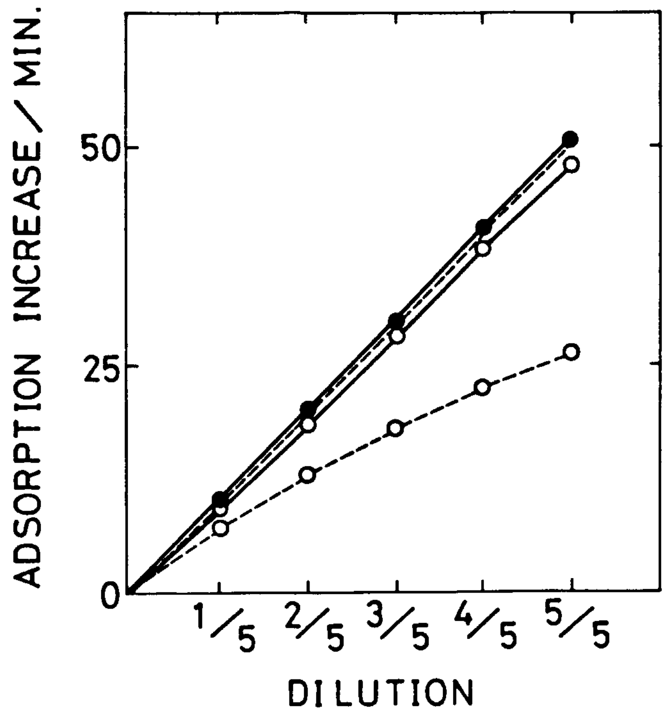
ADSORPTION INCREASE / MIN.
50
25
0
1/5
2/5
3/5
4/5
5/5
DILUTION